# EUROPÄISCHE PATENTSCHRIFT

(11) **EP 1 625 150 B1**
(45) Veröffentlichungstag und Bekanntmachung des Hinweises auf die Patenterteilung: **03.01.2007**
(21) Anmeldenummer: 04731572.6
(22) Anmeldetag: 07.05.2004
(51) Int. Cl.: C07K 5/09

(54) **TRIPEPTIDE UND DEREN DERIVATE FÜR DIE KOSMETISCHE VERWENDUNG ZUR VERBESSERUNG DER HAUTSTRUKTUR**
TRIPEPTIDES AND DERIVATIVES THEREOF FOR COSMETIC APPLICATION IN ORDER TO IMPROVE SKIN STRUCTURE
TRIPEPTIDES ET LEURS DERIVES UTILISES EN COSMETIQUE POUR AMELIORER LA STRUCTURE DE LA PEAU

(30) Priorität: 08.05.2003 CH 807032003
(43) Veröffentlichungstag der Anmeldung: 15.02.2006
(73) Patentinhaber: Pentapharm AG, 4052 Basel (CH)
(72) Erfinder: ZIEGLER, Hugo, CH-4108 Witterswil (CH); HEIDL, Marc, 79639 Grenzach-Wyhlen (DE); IMFELD, Dominik, CH-4142 Münchenstein (CH)
(74) Vertreter: Braun, André jr.
(86) Internationale Anmeldenummer: PCT/CH2004/000278
(87) Internationale Veröffentlichungsnummer: WO 2004/099237

(56) Entgegenhaltungen:
- WO-A-89/12441
- WO-A-02/069884
- FR-A- 2 740 453
- FR-A- 2 810 323
- JP-B- 53 036 477
- US-A1- 2002 051 988

## Beschreibung

### Einleitung:

Es ist bekannt, dass sich die endogene (altersbedingte) oder exogene (lichtbedingte) Alterung in einer irreversiblen Degenerierung der Gewebe, insbesondere der Haut manifestiert. Diese Änderungen entstehen durch eine Reduktion der anabolischen Reaktionen (Synthesen) und eine Erhöhung der katabolischen Reaktionen (Abbau) von Kollagen und Elastin, den zwei Hauptkomponenten der Hautmatrix.

Die Synthese-Reaktionen in der Hautmatrix werden hauptsächlich durch Polypeptide, den sog. Wachstumsfaktoren und Zytokinen, reguliert. Unter diesen Peptiden ist TGFβ1 einer der wichtigsten, in die Synthesereaktionen dieser Hautmatrix involvierten Regulatoren. Er wird in der Matrix durch Keratinozyten und Fibroblasten in latenter Form sezerniert und muss aktiviert werden, um von den Zellrezeptoren erkannt zu werden und eine biologische Antwort (Kollagen- und Elastinsynthese) induzieren zu können. Zwei Formen von latentem TGFβ1 sind vorhanden:
- ein kleiner, latenter Komplex aus 2 TGFβ-Ketten zusammengesetzt, die nicht-kovalent an ein sogenanntes "Latency Associated Protein" (LAP) gebunden sind.
- ein grosser, latenter TGFβ1-Komplex, in dem der kleine, latente TGFβ1-Komplex kovalent (Disulfidbrücke) durch die LAP an ein anderes, sogenanntes "Latent TGFβ Binding Protein" (LTBP) gebunden ist. Es wurde vor kurzem entdeckt, dass in der menschlichen Haut dieser grosse, latente TGFβ1-Komplex an Fibrillin, ein mikrofibrillen-bildendes Molekül, angeschlossen ist; diese Mikrofibrillen sind selber an Elastin gebunden. Der grosse, latente TGFβ1-Komplex bildet also das grösste Reservoir latenter TGFβ1 in der Haut.

Es gibt mehrere physiologische Mechanismen um TGFβ1 zu aktivieren. Das Hauptverfahren in vivo ist die Aktivierung latenter TGFβ1 durch Thrombospondin-1 (TSP-1), ein durch die Hautzellen sezerniertes Protein. Diese Aktivierung beruht auf der Wechselwirkung zwischen dem LAP des latenten TGFβ1 und der Tripeptid-Sequenz RFK (Arg-Phe-Lys) des TSP-1, wo XFX (mit X = basische Aminosäure) die kleinste, zur Aktivierung des latenten TGFβ1 notwendige Sequenz ist.

Während des Alterungsprozesses werden die Bioverfügbarkeit und die TGFβ1-Aktivität durch verminderte genetische Expression und geänderte Kapazität sich an Fibroblasten-Rezeptoren zu fixieren, reduziert. Diese Veränderungen verursachen eine Abschwächung der Synthesereaktionen der Elastin- und Kollagenfasern. Die Abbaureaktionen der Hautmatrix werden hauptsächlich durch proteolytische Enzyme, die Matrixproteinasen (MMPs) provoziert.

Beim chrono-induzierten Alterungsprozess sind die durch die Hautfibroblasten sezernierten MMP1 (oder Kollagenase) und MMP-2 (oder Gelatinase A) involviert. Diese nehmen in der älteren Haut zu und verursachen eine Veränderung der Kollagen- und Elastinfasern. Beim photo-induzierten Alterungsprozess sind die MMP-9 (oder Gelatinase B) und MMP-3 (Leukozytenelastase) involviert. Diese werden durch Keratinozyten und/oder polynukleäre Neutrophile während UV-induzierter inflammatorischer Prozesse sezerniert. Dabei werden die elastischen und Kollagenfasern abgebaut und reduziert (Elastose).

Der verminderte Anabolismus und der erhöhte Katabolismus der Makromoleküle in der Hautmatrix führen also zu einer Gleichgewichtsstörung, die für das Auftreten folgender klinischer Symptome verantwortlich ist: Hautatrophie, Verlust der mechanischen Eigenschaften mit Relief- und Elastizitätsverlust, Hautschlaffheit, tiefe Ausdrucksfalten, beschleunigte Falten- oder Streifenbildung und Verschwinden der natürlichen Hautlinien.

Um die oben beschriebenen Veränderungen und klinischen Symptome zu verhindern, das Aussehen der Hautoberfläche insbesondere durch Reduzieren der Faltentiefe und Fältchenentfernung zu verbessern, wäre es sinnvoll Substanzen zu verwenden, die folgende Effekte gleichzeitig ausüben können:
- Aktivierung der Synthesereaktionen in der Hautmatrix durch Stimulierung der Wachstumsfaktor (TGFβ1)-Aktivität verantwortlich für den Anabolismus der Makromolekülen der extrazellulären Matrix
- Reduzieren der Abbaureaktionen der Hautmatrix durch Modulieren der Metalloproteinasen-Aktivität verantwortlich für den Katabolismus der Makromoleküle in der extrazellulären Matrix und Schutz dieser Komponenten vor der Einwirkung dieser Enzyme

Da das Kollagen ungefähr 80% der Hautproteine ausmacht, ist es einfach zu verstehen, dass die kleinste Verringerung seiner Gewebskonzentration erhebliche Folgen für die mechanischen und physiologischen Eigenschaften der Haut haben kann.

Es wurde nun gefunden, dass es überraschenderweise gelingt, kosmetisch wirksame Tripeptide und Derivate davon (im weiteren "erfindungsgemässe Verbindungen" genannt) und topisch applizierbare, kosmetische Zusammensetzungen zur Bekämpfung chrono- und photo-induzierter Hautalterung (Anti-Aging Mittel) herzustellen, welche schnell und in ausreichender Konzentration durch die Zellmembran zum intrazellulären Wirkort diffundieren können und eine schnelle und starke Stimulierung der Kollagensynthese bewerkstelligen. Dies geschieht durch die Eigenschaft der erfindungsgemässen Verbindungen, die Synthesereaktionen der Hautmatrix durch spezifische Stimulierung des für den Anabolismus der Makromoleküle in der Hautmatrix verantwortlichen Wachstumsfaktors TGFβ1 zu aktivieren.
Diese erfindungsgemässen Verbindungen haben daher einen stimulierenden Einfluss auf die extracelluläre Matrix, welche das mechanische und physiologische Aussehen der Haut entscheidend beeinflussen.
Dabei zeigt sich, dass der Ersatz der mittleren Aminosäure der Tripeptid-Sequenz RFK (Arg-Phe-Lys) des TSP-1 durch eine Aminosäure, welche als Seitenkette eine gegebenenfalls durch Hydroxy substituierte Alkylkette trägt, zusammen mit der Substitution des Peptids mit einer die Penetrationsfähigkeit steigernden lipophilen Gruppe eine schnellere Diffusion durch die Zellmembran zum intrazellulären Wirkort in höherer Konzentration begünstigt und daher diese erfindungsgemässen Verbindungen eine schnellere und stärkere Stimulierung der Kollagensynthese bewirken als die dem Stand der Technik entsprechende und in der Patentanmeldung FR 2810323 am 21.12.01 publizierte Verbindung Elaidyl-Lys-Phe-Lys.

JP 53 936477 B beschreibt das antimikrobiell wirksame modifizierte Tripeptid MOA-Lys-Thr-Lys-Ome (wobei MOA für β-Methoxyacrylat steht).

Die vorliegende Erfindung umfasst Tripeptide und Tripeptid-derivate der allgemeinen Formel I 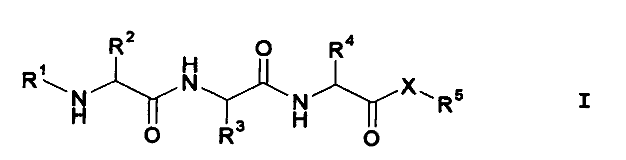 worin
R¹ = H, -C(O)-R⁶, -SO₂-R⁶ oder -C(O)-XR⁶
R² und R⁴ = unabhängig von einander (CH₂)ₙ-NH₂ oder (CH₂)₃-NHC(NH)NH₂,
n = 1-4,
R³ = gegebenenfalls mit Hydroxy substituiertes, lineares oder verzweigtes C₁-C₄-Alkyl,
R⁵ und R⁶ = unabhängig von einander Wasserstoff, gegebenenfalls substituiertes (C₁-C₂₄)-Alkyl, gegebenenfalls substituiertes C₂-C₂₄-Alkenyl, gegebenenfalls substituiertes Phenyl, gegebenenfalls substituiertes Phenyl-C₁-C₄-alkyl oder 9-Fluorenylmethyl, X = Sauerstoff (-O-) oder -NH-; oder
XR⁵ mit X = O auch die Ester von α-Tocopherol, Tocotrienol oder Retinol bedeuten,
mit der Massgabe, dass weder gleichzeitig R¹ und R⁵ Wasserstoff und X Sauerstoff bedeuten, noch gleichzeitig R¹ β-Methoxyacryl, R² und R⁴ (CH₂)₄-NH₂, X Sauerstoff und R⁵ Methyl bedeuten, als Racemate oder in ihrer enantiomerenreinen Form, sowie deren Salze. Diese Verbindungen und Salze eignen sich zur Verwendung als kosmetische Wirkstoffe.

Die oben verwendeten, allgemeinen Ausdrücke sind wie folgt definiert: Unter Alkyl sind sowohl lineare wie verzweigte Alkylgruppen zu verstehen. Beispiele sind Methyl, Ethyl, Propyl, n-Butyl, n-Pentyl, n-Hexyl, n-Heptyl, n-Octyl, n-Nonyl, n-Undecanyl, n-Dodecanyl, n-Tridecanyl, n-Hexadecanyl, n-Heptadecanyl, n-Octadecanyl oder n-Nonadecanyl als unverzweigte und Isopropyl, tert.Butyl, Isobutyl, sec. Butyl, Isoamyl als verzweigte. R⁵ und R⁶ als gegebenenfalls substituiertes Alkyl bedeuten unabhängig voneinander vorzugsweise (C₂-C₂₄)-Alkyl, vorzugsweise (C₃-C₁₈)-Alkyl.

Alkenyl hat die Bedeutung einer ein- oder mehrfach ungesättigten, gegebenenfalls substituierten Alkylgruppe, wie z.B. 8(Z)-Heptadecenyl, 8(Z),11(Z)-Heptadecadienyl, 4(Z),7(Z),10(Z),13(Z)-Nonadecatetraenyl, 8(Z)-11-Hydroxy-octadecenyl. α-Tocopheryl bedeutet (D)-, (L)- oder (DL)-2,5,7,8-Tetramethyl-2-(4',8',12'-trimethyltridecyl)-6-chromanyl, Tocotrienyl ein beliebiges Isomeres von 2,5,7,8-Tetramethyl-2-(4',8',12'-trimethyl-3',7',11'-tridecatrienyl)-6-chromanyl und Retinyl ist 3,7-Dimethyl-9-(2,6,6-trimethyl-1-cyclohexenyl)-2,4,6,8-nonatetraen-1-yl.

Die Verbindungen der Formel (I) können mit Säuren ein- oder mehrwertige, einheitliche oder gemischte Salze bilden, z.B. mit anorganischen Säuren, wie Chlorwasserstoff, Bromwasserstoff, Schwefelsäure oder Phosphorsäure; oder mit geeigneten Carbonsäuren, z.B. aliphatischen Mono- oder Dicarbonsäuren, wie Ameisensäure, Essigsäure, Trifluoressigsäure, Trichloressigsäure, Propionsäure, Glykolsäure, Bernsteinsäure, Fumarsäure, Malonsäure, Maleinsäure, Oxalsäure, Phthalsäure, Zitronensäure, Milchsäure oder Weinsäure; oder mit aromatischen Carbonsäuren, wie Benzoesäure oder Salicylsäure; oder mit aromatisch-aliphatischen Carbonsäuren, wie Mandelsäure oder Zimtsäure; oder mit heteroaromatischen Carbonsäuren, wie Nikotinsäure; oder mit aliphatischen oder aromatischen Sulfonsäuren, wie Methansulfonsäure oder Toluolsulfonsäure. Bevorzugt sind dermatologisch verträgliche Salze. Die allgemeine Formel (I) umfasst alle möglichen isomeren Formen sowie deren Gemische, z.B racemische Gemische und Gemische von Rotameren.

Bevorzugt sind Verbindungen der allgemeinen Formel I, worin
R¹ = -C(O)-R⁶,
R² und R⁴ = unabhängig von einander (CH₂)ₙ-NH₂,
R³ = lineares oder verzweigtes C₁-C₄-Alkyl,
R⁵ = Wasserstoff, (C₁-C₂₄)-Alkyl, (C₂-C₂₄)-Alkenyl, Phenyl, Phenyl-(C₁-C₄)-alkyl, vorzugsweise Wasserstoff, und
R⁶ = (C₁-C₂₄)-Alkyl oder C₂-C₂₄-Alkenyl bedeuten.

Weiter sind die folgenden Verbindungen bevorzugt:
Elaidoyl-Lys-Val-Lys-OH
Elaidoyl-Lys-Thr-Lys-OH
Palm-Lys-Thr-Lys-OH
Palm-Lys-Val-Lys-OH
Palm-Orn-Val-Lys-OH
Palm-Orn-Val-Dab-OH
Palm-Orn-Val-Dap-OH
Palm-Dab-Val-Lys-OH
Palm-Dab-Val-Dab-OH
Palm-Dab-Val-Dap-OH
Palm-Dap-Val-Lys-OH
Palm-Dap-Val-Dab-OH
Palm-Dap-Val-Dap-OH
Palm-Arg-Val-Lys-OH
Palm-Arg-Val-Dab-OH
Palm-Arg-Val-Dap-OH
Palm-Lys-Val-Lys-OH
Palm-Lys-Val-Orn-OH
Palm-Lys-Val-Dab-OH
Palm-Lys-Val-Dap-OH
Palm-Lys-Val-Arg-OH
Palm-Lys-Leu-Lys-OH
Palm-Lys-ILe-Lys-OH
Palm-Lys-ILe-Dab-OH
Palm-Lys-NVa-Dab-OH
Palm-Lys-tBuGly-Dab-OH
Palm-Lys-Leu-Dab-OH
Palm-Lys-ILe-Dap-OH
Palm-Lys-NVa-Dap-OH
Palm-Lys-tBuGly-Dap-OH
Palm-Lys-Leu-Dap-OH
Palm-Lys-NLe-Lys-OH
Palm-Lys-Ala-Lys-OH
Palm-Lys-Ser-Lys-OH
Palm-Lys-HSe-Lys-OH
Palm-Arg-Val-Arg-OH
Pentadecanoyl-Lys-Val-Dab-OH
Pentadecanoyl-Lys-Val-Dap-OH
Heptadecanoyl-Lys-Val-Dab-OH
Heptadecanoyl-Lys-Val-Dap-OH
Myristoyl-Lys-Val-Lys-OH
Myristoyl-Lys-Val-Dab-OH
Myristoyl-Lys-Val-Dap-OH
Lauroyl-Lys-Val-Lys-OH
Caprinoyl-Lys-Val-Lys-OH
Stearoyl-Lys-Val-Lys-OH
Stearoyl-Lys-Val-Dab-OH
Stearoyl-Lys-Val-Dap-OH
Oleolyl-Lys-Val-Lys-OH
Palm-Lys-Val-Dab-OMe
Palm-Lys-Val-Dab-OOctyl
Palm-Lys-Val-Dab-OCetyl
Palm-Lys-Val-Dab-NH₂
Palm-Lys-Val-Dab-NHBu
Palm-Lys-Val-Dab-NHOctyl
Palm-Lys-Val-Dab-NHCetyl
Palm-Lys-Val-Dap-OMe
Palm-Lys-Val-Dap-OOctyl
Palm-Lys-Val-Dap-OCetyl
Palm-Lys-Val-Dap-NH₂
Palm-Lys-Val-Dap-NHBu
Palm-Lys-Val-Dap-NHOctyl
Palm-Lys-Val-Dap-NHCetyl
C₁₄H₂₉-NH-CO-Lys-Val-Lys-OH
C₁₄H₂₉-NH-CO-Dab-Val-Dab-OH
C₁₄H₂₉-NH-CO-Lys-Ile-Dab-OH
C₁₄H₂₉-NH-CO-Lys-Val-Dap-OH
C₁₄H₂₉-NH-CO-Arg-Val-Arg-OH
C₁₄H₂₉-NH-CO-Dab-Val-Dap-OH
C₁₄H₂₉-NH-CO-Lys-Ile-Dap-OH
C₁₄H₂₉-NH-CO-Lys-Val-Dab-OH
C₁₄H₂₉-NH-CO-Dap-Val-Dap-OH
C₁₆H₃₃-NH-CO-Lys-Val-Lys-OH
C₁₈H₃₇-NH-CO-Lys-val-Lys-OH
Boc-Lys-Val-Lys-OH
Ac-Lys-Val-Lys-OH
Z-Lys-Val-Lys-OH
Fmoc-Lys-Val-Lys-OH
C₈H₁₇-SO₂-Lys-Val-Lys-OH
C₁₆H₃₃-SO₂-Lys-Val-Lys-OH
H-Lys-Val-Lys-NH₂
H-Lys-Val-Lys-OOktyl
H-Lys-Val-Lys-OCetyl
H-Lys-Val-Lys-ORetinyl
H-Lys-Val-Lys-OTocopheryl
H-Lys-Val-Lys-OBn
H-Lys-Val-Lys-NH-Cetyl
H-Lys-Val-Lys-NH-Oktyl
H-Lys-Val-Lys-NH-Bn

Die erfindungsgemässen Verbindungen können in Konzentrationen verwendet werden, die zwischen 0,5 und 5.000 ppm (w/w), vorzugsweise zwischen 1 und 1000 ppm (w/w), im kosmetischen Endprodukt variieren. Die erfindungsgemässen Verbindungen können in Form einer Lösung, einer Dispersion, einer Emulsion oder eingekapselt in Träger wie die Makro-, Mikro- oder Nanokapseln, in Liposomen oder Chylomikronen, oder eingeschlossen in Makro-, Mikro- oder Nanoteilchen oder in Mikroschwämme oder absorbiert auf pulverförmigen organischen Polymeren, Talk, Bentonit und anderen mineralischen Trägern verwendet werden.

Die erfindungsgemässen Verbindungen können in jeder galenischen Form verwendet werden: Emulsionen H/E und E/H, Milch, Lotionen, Salben, gelierende und viskose, spannungsaktive und emulgierende Polymere, Pommaden, Shampoos, Seifen, Gele, Puder, Sticks und Stifte, Sprays, Körperöle, Gesichtsmasken, Pflaster.

Die erfindungsgemässen Verbindungen sowie die kosmetischen Zusammensetzungen, die sie enthalten, werden für Hautpflegeprodukte eingesetzt, insbesondere gegen die Bildung und die Verschlimmerung der Falten und gegen alle Folgen der Hautalterung natürlicher oder beschleunigter Art (Heliodermie, Verschmutzung)

Ein weiterer Aspekt der vorliegenden Erfindung sind die folgenden Zusammensetzungen:

Die Tripeptid-Derivate mit der allgemeinen Formel (I) können mit mindestens einem zusätzlichen Hautpflegewirkstoff gemischt werden. Diese Zusammensetzungen können ausserdem weitere dermatologisch annehmbare Träger enthalten.

Zusätzliche Hautpflegewirkstoffe: In einer bevorzugten Ausführungsform, in der die Zusammensetzung in Kontakt mit menschlichem Horngewebe sein soll, sollten die zusätzlichen Komponenten zur Auftragung auf Horngewebe geeignet sein, das bedeutet, dass sie bei Einbringung in die Zusammensetzung zur Verwendung in Kontakt mit menschlichem Horngewebe geeignet sind, ohne innerhalb des Bereichs vernünftiger medizinischer Beurteilung ungeeignete Toxizität, Unverträglichkeit, Instabilität, allergische Reaktion und dergleichen zu ergeben. Das CTFA Cosmetic Ingredient Handbook, 2. Auflage (1992) beschreibt eine weite Vielfalt nicht einschränkender kosmetischer und pharmazeutischer Bestandteile, die üblicherweise in der Hautpflegeindustrie verwendet werden, die zur Verwendung in den erfindungsgemässen Zusammensetzungen geeignet sind. Beispiele für diese Klassen von Bestandteilen schliessen ein: Schleifmittel, Absorbentien, ästhetische Komponenten wie Duftstoffe, Pigmente/Färbungsmittel/Farbmittel, etherische Öle, Sinneswahrnehmungsmittel für die Haut, Adstringentien, usw. (z. B. Nelkenöl, Menthol, Kampfer, Eukalyptusöl, Eugenol, Menthyllactat, Hamamelis-Destillat), Anti-Aknemittel, Mittel gegen Zusammenklumpen, Antischaummittel, antimikrobielle Mittel (z. B. Iodpropinylbutylcarbamat), Antioxidantien, Bindemittel, biologische Additive, Pufferungsmittel, Füllmittel, Chelatbildner, chemische Additive, Färbungsmittel, kosmetische Adstringentien, kosmetische Biozide, Denaturierungsmittel, medizinische Adstringentien, externe Analgetika, Filmbildner oder -materialien, z. B. Polymere zur Unterstützung der Filmbildungseigenschaften und Substantivität der Zusammensetzung (z. B. Copolymer von Eicosen und Vinylpyrrolidon), Opazifizierungsmittel, Mittel zur Einstellung des pH-Werts, Treibmittel, Reduktionsmittel, Sequestrierungsmittel, Hautbleich- und -aufhellungsmittel (z. B. Alpha- oder Beta-Arbutin, Hydrochinon, Kojisäure, Ascorbinsäure, Magnesiumascorbylphosphat, Ascorbylglucosamin), Hautkonditionierungsmittel (z.B. Feuchthaltemittel einschliesslich diverser und okklusiver), Hautberuhigungs- oder -heilungsmittel (z. B. Panthenol und Derivate (z. B. Ethylpanthenol), Aloe vera, Panthothensäure und ihre Derivate, Allantoin, Bisabolol und Dikaliumglycyrrhizinat), Hautbehandlungsmittel, Verdickungsmittel und Vitamine und Derivate davon.

In jeder Ausführungsform der vorliegenden Erfindung können die hier anwendbaren Wirkstoffe jedoch nach dem Nutzen, den sie bieten, oder ihrem postulierten Wirkmodus kategorisiert werden. Es sei jedoch darauf hingewiesen, dass die hier anwendbaren Wirkstoffe in manchen Fällen mehr als einen Nutzen liefern können oder nach mehr als einem Wirkmodus wirken können. Die Klassifizierungen erfolgen hier daher der Bequemlichkeit halber und sollen den Wirkstoff nicht auf diese spezielle aufgeführte Anwendung oder diese speziellen aufgeführten Anwendungen begrenzen.

Farnesol: Die erfindungsgemässen topischen Zusammensetzungen können eine sichere und wirksame Menge Farnesol enthalten. Farnesol ist eine natürlich vorkommende Substanz, die vermutlich als Vorläufer und/oder Zwischenstufe in der Biosynthese von Squalen und Sterolen wirkt, insbesondere Cholesterol. Farnesol ist auch an Proteinmodifizierung und -regulierung beteiligt (z. B. Farnesylierung von Proteinen), und es gibt einen Zellkernrezeptor, der auf Farnesol anspricht.

Chemisch ist Farnesol [2E,6E]-3,7,11-Trimethyl-2,6,10-dodecatrien-1-ol, und "Farnesol" wie hier verwendet schliesst Isomere und Tautomere davon ein. Farnesol ist kommerziell erhältlich, z. B. unter den Namen Farnesol (eine Mischung von Isomeren von Dragoco, 10 Gordon Drive, Totowa, N.J., USA) und trans-trans-Farnesol (Sigma Chemical Company, P. O. Box 14508, St. Louis, Mo., USA).

Phytantriol: Die erfindungsgemässen topischen Zusammensetzungen können eine sichere und wirksame Menge Phytantriol enthalten. Phytantriol ist der übliche Name für die Chemikalie, die als 3,7,11,15-Tetramethylhexadecan-1,2,3-triol bekannt ist. Phytantriol ist beispielsweise als Reparaturmittel für Spinnenmale/Rotfleckigkeit der Haut, Reparaturmittel für dunkle Augenringe/geschwollene Augen, Reparaturmittel für Blässe, Reparaturmittel für schlaffe Haut, Mittel gegen Juckreiz, Hautverdickungsmittel, Porenverkleinerungsmittel, Mittel zur Verringerung von Öligkeit/Glanz, Reparaturmittel für post-entzündliche Hyperpigmentierung, Wundbehandlungsmittel, Anti-Cellulitismittel und Mittel zur Regulierung der Oberflächenbeschaffenheit der Haut, einschliesslich Fältchen und feinen Linen, brauchbar.

Wirkstoffe der Desquamation: Eine sichere und wirksame Menge eines Wirkstoffs der Desquamation kann den erfindungsgemässen Zusammensetzungen zugefügt werden, insbesondere etwa 0,1 % bis etwa 10 %, bevorzugter etwa 0,2 % bis etwa 5 %, auch bevorzugt etwa 0,5 % bis etwa 4 %, bezogen auf das Gewicht der Zusammensetzung. Wirkstoffe, welche die Desquamation beeinflussen, verstärken die positiven Wirkungen der vorliegenden Erfindung auf das Aussehen der Haut. Die Desquamationswirkstoffe neigen beispielsweise dazu, die Oberflächenbeschaffenheit (z. B. Glattheit) der Haut zu verbessern. Ein zur hiesigen Verwendung geeignetes System enthält Sulfhydrylverbindungen und zwitterionische Tenside und ist in der US-A-5,681,852 von Bissett beschrieben, hier zitiert zum Zweck der Bezugnahme. Ein anderes zur hiesigen Verwendung geeignetes System enthält Salicylsäure und zwitterionische Tenside und ist in der US-A-5,652,228 von Bissett beschrieben, hier zitiert zum Zweck der Bezugnahme. Zwitterionische Tenside, wie sie in diesen Anmeldungen beschrieben sind, sind hier auch als Desquamationsmittel brauchbar, wobei Cetylbetain besonders bevorzugt ist.

Anti-Aknewirkstoffe: Die erfindungsgemässen Zusammensetzungen können eine sichere und wirksame Menge von einem oder mehreren Anti-Aknewirkstoffen enthalten. Beispiele für solche Anti-Aknewirkstoffe schliessen Resorcin, Schwefel, Salicylsäure, Benzoylperoxid, Erythromycin, Zink, usw. ein. Weitere Beispiele für geeignete Anti-Aknewirkstoffe sind detaillierter in US-A-5,607,980 beschrieben, erteilt an McAtee am 4. März 1997.

Anti-Faltenwirkstoffe/Anti-Atrophiewirkstoffe: Die erfindungsgemässen Zusammensetzung können ferner eine sichere und wirksame Menge von einem oder mehreren Anti-Faltenwirkstoffen oder Anti-Atrophiewirkstoffen enthalten. Beispielhafte Anti-Falten/Anti-Atrophie-Wirkstoffe, die zur Verwendung in den erfindungsgemässen Zusammensetzungen geeignet sind, schliessen schwefelhaltige D- und L-Aminosäuren und ihre Derivate und Salze, insbesondere die N-Acetylderivate, wobei ein bevorzugtes Beispiel hierfür N-Acetyl-L-cystein ist; Thiole, z. B. Ethanthiol; Hydroxysäuren (z. B. α-Hydroxysäuren wie Milchsäure und Glykolsäure oder β-Hydroxysäuren wie Salicylsäure und Salicylsäurederivate, wie die Octanoylderivate), Phytinsäure, Liponsäure; Lysophosphatidinsäure, Haut-Peelingmittel (z. B. Phenol und dergleichen), Vitamin B₃-Verbindungen und Retinoide ein, die die Vorteile der vorliegenden Erfindung für das Aussehen von Horngeweben verbessern, insbesondere bei der Regulierung des Horngewebezustands, z. B. Hautzustands.
a) Vitamin B3-Verbindungen: Die erfindungsgemässen Zusammensetzungen können eine sichere und wirksame Menge einer Vitamin B3-Verbindung enthalten. Vitamin B3-Verbindungen sind besonders nützlich zur Regulierung des Hautzustands, wie in der gleichzeitig anhängigen US-Patentanmeldung mit dem Aktenzeichen Nr. 08/834,010 beschrieben ist, eingereicht am 11. April 1997 (entsprechend der internationalen Veröffentlichung WO 97/39733 A1, veröffentlicht am 30. Oktober 1997). Beispielhafte Derivate der genannten Vitamin B₃-Verbindungen schliessen Nicotinsäureester einschliesslich nicht-vasodilatierender Ester von Nicotinsäure (z.B. Tocopherylnicotinat), Nicotinylaminosäuren, Nicotinylalkoholestern von Carbonsäuren, Nicotinsäure-N-oxid und Niacinamid-N-oxid ein.
b) Retinoide: Die erfindungsgemässen Zusammensetzungen können auch ein Retinoid enthalten. "Retinoid" schliesst, wie hier verwendet, alle natürlichen und/oder synthetischen Analoga von Vitamin A oder retinolartigen Verbindungen ein, die die biologische Wirksamkeit von Vitamin A in der Haut besitzen, sowie die geometrischen Isomere und Stereoisomere dieser Verbindungen. Das Retinoid ist vorzugsweise Retinol, Retinolester (z. B. C₂- bis C₂₂-Alkylester von Retinol einschliesslich Retinylpalmitat, Retinylacetat, Retinylpropionat), Retinal und/oder Retinsäure (einschliesslich all-trans-Retinsäure und/oder 13-cis-Retinsäure), insbesondere von Retinsäure verschiedene Retinoide. Andere geeignete Retinoide sind Tocopherylretinoat [Tocopherolester von Retinsäure (trans oder cis)], Adaptalen {6-[3-(1-Adamantyl)-4-methoxyphenyl]-2-naphthoesäure} und Tazaroten (Ethyl-6-[2-(4,4-dimethylthiochroman-6-yl)-ethinyl]nicotinat). Bevorzugte Retinoide sind Retinol, Retinylpalmitat, Retinylacetat, Retinylpropionat, Retinal und Kombinationen davon. Die erfindungsgemässen Zusammensetzungen können eine sichere und wirksame Menge des Retinoids enthalten, so dass die resultierende Zusammensetzung sicher und wirksam zur Regulierung des Zustands von Horngewebe ist, vorzugsweise zur Regulierung von sichtbaren und/oder fühlbaren Diskontinuitäten der Haut, insbesondere zur Regulierung von Zeichen der Hautalterung, bevorzugter zur Regulierung von sichtbaren und/oder fühlbaren Diskontinuitäten der Hautoberflächenbeschaffenheit, die mit Hautalterung zusammenhängen.
(c) Hydroxysäuren: Die erfindungsgemässen Zusammensetzungen können eine sichere und wirksame Menge einer Hydroxysäure enthalten. Bevorzugte Hydroxysäuren zur Verwendung in den erfindungsgemässen Zusammensetzungen schliessen Salicylsäure und Salicylsäurederivate ein.

Peptide: Zusätzliche Peptide einschliesslich, aber nicht begrenzt auf Di-, Tri-, Tetra- und Pentapeptide und Derivate davon können den erfindungsgemässen Zusammensetzungen in sicheren und wirksamen Mengen zugefügt werden. "Peptide" bezieht sich hier auf sowohl die natürlich vorkommenden Peptide als auch die synthetisierten Peptide und umfasst auch Peptidmimetika und metallkomplexe von "Peptiden". Die natürlich vorkommenden und im Handel erhältlichen Zusammensetzungen, die Peptide enthalten, sind hier auch brauchbar.

Zur hiesigen Verwendung geeignete Dipeptide schliessen Carnosin (β-Ala-His) ein. Zur hiesigen Verwendung geeignete Tripeptide schliessen Gly-His-Lys, Arg-Lys-Arg, His-Gly-Gly ein. Bevorzugte Tripeptide und Derivate davon schliessen Palmitoyl-Gly-His-Lys, das als Biopeptid CL^{™} erworben werden kann (100 ppm Palmitoyl-Gly-His-Lys, kommerziell erhältlich von Sederma, Frankreich), Peptid CK (Arg-Lys-Arg), Peptid CK+ (ac-Arg-Lys-Arg-NH₂) und einen Kupferkomplex von Gly-His-Lys oder von His-Gly-Gly ein, das kommerziell als Lamin von Sigma (St. Louis, Mo., USA) erhältlich ist. Zur hiesigen Verwendung geeignete Tetrapeptide schliessen Peptid E ein, Arg-Ser-Arg-Lys. Beispiele für Pentapeptide sind Matrixyl (Palmitoyl-Lys-Thr-Thr-Lys-Ser), erhältlich von Sederma, Frankreich, und jene, die in WO 03/037933 (Pentapharm, Schweiz) beschrieben sind.

Antioxidantien/ Radikal fänger: Die erfindungsgemässen Zusammensetzungen können eine sichere und wirksame Menge eines Antioxidans/ Radikalfängers einschliessen. Das Antioxidans/ Radikalfänger ist besonders geeignet zur Bereitstellung von Schutz vor UV-Strahlung, die zu verstärktem Abschuppen oder Oberflächenstrukturveränderungen im Stratum corneum führen kann, und anderen Umweltfaktoren, die zu Hautschäden führen können.

Antioxidantien/ Radikalfänger wie Ascorbinsäure (Vitamin C) und deren Salze, Ascorbylester von Fettsäuren, Ascorbinsäurederivate (z. B. Magnesiumascorbylphosphat, Natriumascorbylphosphat, Ascorbylsorbat), Tocopherol (Vitamin E), Tocopherolsorbat, Tocopherolacetat, andere Ester von Tocopherol, butylierte Hydroxybenzoesäuren und ihre Salze, 6-Hydroxy-2,5,7,8-tetramethylchroman-2-carbonsäure (kommerziell unter dem Handelsnamen Trolox^{™} erhältlich), Gallussäure und ihre Alkylester, insbesondere Propylgallat, Harnsäure und ihre Salze und Alkylester, Sorbinsäure und ihre Salze, Liponsäure, Amine (z. B. N,N-Diethylhydroxylamin, Aminoguanidin), Sulfhydrylverbindungen (z.B. Glutathion), Dihydroxyfumarsäure und ihre Salze, Lycinpidolat, Argininpidolat, Nordihydroguaiaretinsäure, Bioflavonoide, Curcumin, Lysin, 1-Methionin, Prolin, Peroxid-dismutase, Silymarin, Teeextrakte, Traubenhaut/Kern-Extrakte, Melanin und Rosmarinextrakte, können verwendet werden. Bevorzugte Antioxidantien/ Radikalfänger sind ausgewählt aus Tocopherolsorbat und anderen Estern von Tocopherol, insbesondere Tocopherolsorbat. Die Verwendung von Tocopherolsorbat in topischen Zusammensetzung, die in der vorliegenden Erfindung verwendbar sind, ist beispielsweise in US-A-4,847,071 beschrieben, erteilt am 11. Juli 1989 an Donald L. Bissett, Rodney D. Bush und Ranjit Chatterjee.

Chelatbildner: Die erfindungsgemässen Zusammensetzungen können auch eine sichere und wirksame Menge eines Chelatbildners oder Chelierungsmittels enthalten. "Chelatbildner" oder "Chelierungsmittel" bezeichnen hier einen Wirkstoff, der in der Lage ist, ein Metallion aus einem System durch Bildung eines Komplexes zu entfernen, so dass das Metallion nicht leicht an chemischen Reaktionen teilnehmen kann oder diese katalysieren kann. Der Einschluss eines Chelierungsmittels ist besonders nützlich, um Schutz vor UV-Strahlung, die zu übermässigem Abschuppen oder Hautoberflächenstrukturänderungen beitragen kann, oder anderen Umweltfaktoren zu liefern, die zu Hautschäden führen können.

Beispielhafte Chelatbildner, die hier brauchbar sind, sind in US-A-5,487,884, erteilt am 30. Januar 1996 an Bissett et al., der Internationalen Veröffentlichung Nr. 91/16035, Bush et al., veröffentlicht am 31. Oktober 1995, und der Internationalen Veröffentlichung Nr. 91/16034, Bush et al., veröffentlicht am 31. Oktober 1995 offenbart. Bevorzugte Chelatbildner, die in den erfindungsgemässen Zusammensetzungen brauchbar sind, sind Furildioxim, Furilmonoxim und Derivate davon.

Flavonoide: Die erfindungsgemässen Zusammensetzungen können gegebenenfalls eine Flavonoidverbindung enthalten. Flavonoide sind umfassend in US-A-5,686,082 und US-A-5,686,367 offenbart, die hier beide zum Zweck der Bezugnahme zitiert werden. Zur erfindungsgemässen Verwendung geeignete Flavonoide sind Flavanone ausgewählt aus unsubstituierten Flavanonen, monosubstituierten Flavanonen und Mischungen davon; Chalconen ausgewählt aus unsubstituierten Chalconen, monosubstituierten Chalconen, disubstituierten Chalconen, trisubstituierten Chalconen und Mischungen davon; Flavonen ausgewählt aus unsubstituierten Flavonen, monosubstituierten Flavonen, disubstituierten Flavonen und Mischungen davon, einem oder mehreren Isoflavonen; Coumarinen ausgewählt aus unsubstituierten Coumarinen, monosubstituierten Coumarinen, disubstituierten Coumarinen und Mischungen davon; Chromonen ausgewählt aus unsubstituierten Chromonen, monosubstituierten Chromonen, disubstituierten Chromonen und Mischungen davon; einem oder mehreren Dicoumarolen; einem oder mehreren Chromanonen; einem oder mehreren Chromanolen; Isomeren (z. B. cis/trans-Isomeren) davon und Mischungen davon. Mit dem Begriff "substituiert" werden hier Flavonoide bezeichnet, bei denen ein oder mehrere Wasserstoffatom(e) unabhängig durch Hydroxyl, C₁- bis C₈-Alkyl, C₁- bis C₄-Alkoxyl, O-Glycosid und dergleichen oder eine Mischung dieser Substituenten ersetzt worden sind.

Beispiele für geeignete Flavonoide schliessen unsubstituiertes Flavanon, Monohydroxyflavanone (z. B. 2'-Hydroxyflavanon, 6-Hydroxyflavanon, 7-Hydroxyflavanon, usw.), Monoalkoxyflavanone (z. B. 5-Methoxyflavanon, 6-Methoxyflavanon, 7-Methoxyflavanon, 4'-Methoxyflavanon, usw.), unsubstituiertes Chalcon (insbesondere unsubstituiertes trans-Chalcon), Monohydroxychalcone (z. B. 2'-Hydroxychalcon, 4'-Hydroxychalcon, usw.), Dihydroxychalcone (z. B. 2',4-Dihydroxychalcon, 2',9'-Dihydroxychaslcon, 2,2'-Dihydroxychalcon, 2',3-Dihydroxychalcon, 2',5'-Dihydroxychalcon, usw.) und Trihydroxychalcone (z. B. 2',3',4'-Trihydroxychalcon, 4,2',4'-Trihydroxychalcon, 2,2',4'-Trihydroxychalcone, usw.), unsubstituiertes Flavon, 7,2'-Dihydroxyflavon, 3',9'-Dihydroxynaphthoflavon, 4'-Hydroxyflavon, 5,6-Benzoflavon und 7,8-Benzoflavon, unsubstituiertes Isoflavon, Daidzein (7,4'-Dihydroxyisoflavon), 5,7-Dihydroxy-4'-methoxyisoflavon, Soja-Isoflavone (eine aus Soja extrahierte Mischung), unsubstituiertes Coumarin, 4-Hydroxycoumarin, 7-Hydroxycoumarin, 6-Hydroxy-4-methylcoumarin, unsubstituiertes Chromon, 3-Formylchromon, 3-Formyl-6-isopropylchromon, unsubstituiertes Dicoumarol, unsubstituiertes Chromanon, unsubstituiertes Chromanol und Mischungen davon ein, sind jedoch nicht auf diese begrenzt.

Hier sind zur Verwendung unsubstituiertes Flavanon, Methoxyflavanone, unsubstituiertes Chalcon, 2',9'-Dihydroxychalcon und Mischungen davon bevorzugt. Besonders bevorzugt sind unsubstituiertes Flavanon, unsubstituiertes Chalcon (insbesondere das trans-Isomer) und Mischungen davon.

Entzündungshemmende Mittel: Das entzündungshemmende Mittel erhöht die Vorteile der vorliegenden Erfindung für das Aussehen der Haut, z.B. tragen solche Mittel zu einer einheitlicheren und annehmbaren Hauttönung oder -farbe bei.

Steroidale entzündungshemmende Mittel einschliesslich Corticosteroiden wie Hydrocortison, Hydroxyltriamcinolon, α-Methyldexamethason, Dexamethasonphosphat, Beclomethasondipropionat, Clobetasolvalerat, Desonid, Desoxymethason, Desoxycorticosteronacetat, Dexamethason, Dichlorison, Diflorasondiacetat, Diflucortolonvalerat, Fluadrenolon, Fluclorolonacetonid, Fludrocortison, Flumethasonpivalat, Fluosinolonacetonid, Fluocinonid, Flucortinbutylester, Fluocortolon, Flupredniden (Fluprednyliden)-acetat, Flurandrenolon, Halcinonid, Hydrocortisonacetat, Hydrocortisonbutyrat, Methylprednisolon, Triamcinolonacetonid, Cortison, Cortodoxon, Flucetonid, Fludrocortison, Difluorosondiacetat, Fluradrenolon, Fludrocortison, Diflurosondiacetat, Fluradrenolonacetonid, Medryson, Amcinafel, Amcinafid, Betamethason und die restlichen seiner Ester, Chloroprednison, Chlorprednisonacetat, Clocortelon, Clescinolon, Dichlorison, Diflurprednat, Flucloronid, Flunisolid, Fluoromethalon, Fluperolon, Fluprednisolon, Hydrocortisonvalerat, Hydrocortisoncyclopentylpropionat, Hydrocortamat, Meprednison, Paramethason, Prednisolon, Prednison, Beclomethasondipropionat, Triamcinolon und Mischungen davon können verwendet werden, sind jedoch nicht darauf begrenzt. Das zur Verwendung bevorzugte steroidale antientzündliche Mittel ist Hydrocortison.

Eine zweite Klasse entzündungshemmender Mittel, die in den Zusammensetzungen verwendet werden können, schliesst die nicht-steroidalen entzündungshemmenden Mittel ein. Die Vielfalt von Verbindungen, die diese Gruppe einschliesst, sind Fachleuten wohl bekannt. Hinsichtlich detaillierter Offenbarung der chemischen Struktur, Synthese, Nebenwirkungen, usw. von nicht-steroidalen entzündungshemmenden Mitteln kann auf die Standardlehrbücher verwiesen werden, einschliesslich Anti-inflammatory and Anti-Rheumatic Drugs, K. D. Rainsford, Band I bis III, CRC Press, Boca Raton (1985) und Anti-inflammatory Agents, Chemistry and Pharmacology, 1, R.A.Scherrer et al., Acad. Press, New York (1974).

Spezielle nicht-steroidale entzündungshemmende Mittel, die in den erfindungsgemässen Zusammensetzungen brauchbar sind, schliessen die folgenden ein, sind jedoch nicht auf diese begrenzt:
1) die Oxicame, wie Piroxicam, Isoxicam, Tenoxicam, Sudoxicam und CP-14,304;
2) die Salicylate, wie Aspirin, Disalcid, Benorylat, Trilisat, Safapryn, Solprin, Diflunisal und Fendosal;
3) die Essigsäurederivate, wie Diclofenac, Fenclofenac, Indomethacin, Sulindac, Tolmetin, Isoxepac, Furofenac, Tiopinac, Zidometacin, Acematacin, Fentiazac, Zomepirac, Clindanac, Oxepinac, Felbinac und Ketorolac;
4) die Fenamate, wie Mefenamin-, Meclofenamin-, Flufenamin-, Niflumin- und Tolfenaminsäuren;
5) die Propionsäurederivate, wie Ibuprofen, Naproxen, Benoxaprofen, Flurbiprofen, Ketoprofen, Fenoprofen, Fenbufen, Indopropfen, Pirprofen, Carprofen, Oxaprozin, Pranoprofen, Miroprofen, Tioxaprofen, Suprofen, Alminoprofen und Tiaprofensäure und
6) die Pyrazole, wie Phenylbutazon, Oxyphenbutazon, Feprazon, Azapropazon und Trimethazon.

Es können auch Mischungen dieser nicht-steroidalen entzündungshemmenden Mittel verwendet werden, sowie die dermatologisch annehmbaren Salze und Ester dieser Mittel. Etofenamat, ein Flufenaminsäurederivat, ist beispielsweise zur topischen Anwendung besonders nützlich. Von den nicht-steroidalen entzündungshemmenden Mitteln sind Ibuprofen, Naptoxen, Flufenaminsäure, Etofenamat, Aspirin, Mefenaminsäure, Meclofenaminsäure, Piroxicam und Felbinac bevorzugt; Ibuprofen, Naproxen, Ketoprofen, Etofenamat, Aspirin und Flufenaminsäure sind besonders bevorzugt.

Schliesslich sind die sogenannten "natürlichen" entzündungshemmenden Mittel in den erfindungsgemässen Verfahren nützlich. Solche Mittel können geeigneterweise als Extrakt durch geeignete physikalische und/oder chemische Isolierung aus natürlichen Quellen erhalten werden (z. B. Pflanzen, Pilze, Nebenprodukte von Mikroorganismen) oder können synthetisch hergestellt werden. Beispielsweise können Candelillawachs, Bisabolol (z. B. α-Bisabolol), Aloe vera, Pflanzensterole (z. B. Phytostyrol), Manjistha (aus Pflanzen der Gattung Rubia extrahiert, insbesondere Rubia Cordifolia) und Guttal (aus Pflanzen der Gattung Commiphora extrahiert, insbesondere Commiphora Mukul), Kolaextrakt, Chamomilla (echte Kamille), Rotkleeextrakt und Meeresalgenextrakt verwendet werden.

Hier brauchbare zusätzliche antientzündliche Mittel schliessen Verbindungen der Lakritze (der Familie der Pflanzengattung/-spezies Glycyrrhiza glabra) einschliesslich Glycyrrhetsäure, Glycyrrhizinsäure und Derivaten davon (z. B. Salze und Ester) ein. Geeignete Salze der genannten Verbindungen schliessen Metall- und Ammoniumsalze ein.

Anti-Cellulitismittel: Die erfindungsgemässen Zusammensetzungen können auch eine sichere und wirksame Menge eines Anti-Cellulitismittels enthalten. Geeignete Mittel schliessen Xanthinverbindungen (z. B. Coffein, Theophyllin, Theobromin und Aminophyllin) ein, sind jedoch nicht auf diese begrenzt.

Hautbräunungsmittel: Dihydroxyaceton, das auch als DHA oder 1,3-Dihydroxy-2-propanon bekannt ist, ist ein weisses bis schmutzigweisses kristallines Pulver. Die Verbindung kann als Mischung von Monomeren und Dimeren vorliegen, wobei die Dimere im festen kristallinen Zustand überwiegen. Beim Erwärmen oder Schmelzen werden die Dimere unter Bildung der Monomere gespalten. Diese Umwandlung der dimeren Form in die monomere Form findet auch in wässriger Lösung statt. Ein weiteres Beispiel ist Erythrulose, erhältlich von Pentapharm, Schweiz. DHA und Erythrulose können in Kombination verabreicht werden.

Hautaufhellungsmittel: Geeignete Hautaufhellungsmittel schliessen jene ein, die in der Technik bekannt sind, einschliesslich Kojisäure, Arbutin, α-Arbutin, Ascorbinsäure und Derivaten davon (z. B. Magnesiumascorbylphosphat oder Natriumascorbylphosphat) und Extrakten (z. B. Maulbeerextrakt, Placentaextrakt).

Hautberuhigungs- und Hautheilungswirkstoffe: Die erfindungsgemässen Zusammensetzungen können einen Hautberuhigungs- oder Hautheilungswirkstoff umfassen. Hautberuhigungs- oder Hautheilungswirkstoffe, die zur hiesigen Verwendung geeignet sind, schliessen Panthothensäurederivate (einschliesslich Panthenol, Dexpanthenol, Ethylpanthenol), Aloe vera, Allantoin, Bisabolol und Dikaliumglycyrrhizinat ein.

Bisabolol: Die erfindungsgemässen topischen Zusammensetzungen können auch eine sichere und wirksame Menge Bisabolol enthalten. Bisabolol ist ein natürlich vorkommender, ungesättigter, monocyclischer Terpenalkohol mit der folgenden Struktur:

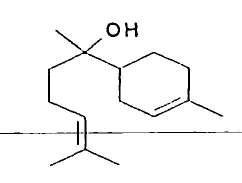

Es ist die Hauptwirkkomponente von Kamillenextrakt/-öl. Bisabolol kann synthetischer (d,1-α-Isomer oder (+/-)-α-Isomer) oder natürlicher ((-)-α-Isomer) Herkunft sein und kann als im Wesentlichen reine Verbindungen oder Mischungen von Verbindungen verwendet werden (z. B. Extrakte aus natürlichen Quellen, wie Kamille). Die α-Form von Bisabolol (a-Bisabolol) wird in einer Vielfalt von Kosmetikprodukte als Hautkonditionierung- oder -beruhigungsmittel verwendet. "Bisabolol" soll hier Kamillenextrakt oder -öl und beliebige Isomere und Tautomere von diesen einschliessen. Geeignete Bisabololverbindungen sind kommerziell als natürliches Material von Dragoco (Totowa, N. J., USA) unter dem Produktnamen α-Bisabolol natural und als synthetisches Material von Fluka (Milwaukee, Wis., USA) unter dem Produktnamen α-Bisabolol erhältlich.

Antimikrobielle und antimykotische Wirkstoffe: Die erfindungsgemässen Zusammensetzungen können einen antimikrobiellen oder antimykotische Wirkstoff enthalten. Solche Wirkstoffe sind in der Lage, Mikroben zu zerstören, die Entwicklung von Mikroben zu verhindern oder die pathogene Wirkung von Mikroben zu verhindern. Bevorzugte Beispiele für hier brauchbare Wirkstoffe schliessen jene ausgewählt aus Salicylsäure, Benzoylperoxid, 3-Hydroxybenzoesäure, Glykolsäure, Milchsäure, 4-Hydroxybenzoesäure, Acetylsalicylsäure, 2-Hydroxybutansäure, 2-Hydroxypentansäure, 2-Hydroxyhexansäure, cis-Retinsäure, trans-Retinsäure, Retinol, Phytinsäure, N-Acetyl-L-cystein, Lipoinsäure, Azelainsäure, Arachidonsäure, Benzoylperoxid, Tetracyclin, Ibuprofen, Naproxen, Hydrocortison, Acetominophen, Resorcin, Phenoxyethanol, Phenoxypropanol, 2,4,4'-Trichlor-2'-hydroxydiphenylether, 3,4,4'-Trichlorcarbanilid, Octopirox, Lidocainhydrochlorid, Clotrimazol, Miconazol, Ketoconazol, Neocycinsulfat und Mischungen davon ein.

Sonnenschutzwirkstoffe: Die Einwirkung von ultraviolettem Licht kann zu übermässiger Abschuppung und Änderungen der Oberflächenstruktur im Stratum corneum führen. Daher können die erfindungsgemässen Zusammensetzungen gegebenenfalls einen Sonnenstoffwirkstoff enthalten. "Sonnenschutzwirkstoff" schliesst hier sowohl Sonnenschutzmittel als auch physikalische Sunblocker ein. Geeignete Sonnenschutzwirkstoffe können organisch oder anorganisch sein.

Hier brauchbare anorganische Sonnenschutzmittel schliessen die folgenden Metalloxide ein: Titandioxid, Zinkoxid, Zirkoniumoxid, Eisenoxid und Mischungen davon.

Beispiele für organische Sonnenschutzmittel sind 2-Ethylhexyl-p-methoxycinnamat (kommerziell erhältlich als PARSOL MCX), 4,4'-t-Butylmethoxydibenzoyl-methan (kommerziell erhältlich als PARSOL 1789), 2-Hydroxy-4-methoxybenzophenon, Octyldimethyl-p-aminobenzoesäure, Digalloyltrioleat, 2,2-Dihydroxy-4-methoxybenzophenon, Ethyl-4-(bis(hydroxypropyl))aminobenzoat, 2-Ethylhexyl-2-cyano-3,3-diphenylacrylat, 2-Ethylhexylsalicylat, Glyceryl-p-aminobenzoat, 3,3,5-Trimethylcyclohexylsalicylat, Methylanthranilat, p-Dimethylaminobenzoesäure oder Aminobenzoat, 2-Ethylhexyl-p-dimethylaminobenzoat, 2-Phenylbenzimidazol-5-sulfonsäure, 2-(p-Dimethylaminophenyl)-5-sulfonbenzoxazonsäure, Octocrylen, 2,2'-Methylen-bis-[6-(2H-benzotriazol-2-yl)-4-(1,1,3,3-tetramethylbutyl)-phenol] erhältlich von Ciba SC als TINOSORB^{™} M, 2,4-Bis-[(4-(2-ethylhexyloxy)-2-hydroxy)-phenyl]-6-(4-methoxyphenyl)-1,3,5-triazin, erhältlich von Ciba SC als TINOSORB^{™} S und Mischungen dieser Verbindungen.

In den Zusammensetzungen sind auch Sonnenschutzmittel besonders brauchbar, wie sie in US-A-4,937,370, erteilt an Sabatelli am 26. Juni 1990, und US-A-4,999,186, erteilt an Sabatelli&Spirnak am 12. März 1991, offenbart sind. Die dort offenbarten Sonnenschutzmittel haben zwei eigene chromophore Gruppen in einem einzigen Molekül, die unterschiedliche Ultraviolettstrahlungsabsorptionsspektren zeigen. Eine der chromophoren Gruppen absorbiert vorwiegend im UV-B-Strahlungsbereich, und die andere absorbiert stark im UV-A-Strahlungsbereich.

Bevorzugte Verbindungen dieser Klasse von Sonnenschutzmitteln sind 4-N,N-(2-Ethylhexyl)methyl-aminobenzoesäureester von 2,4-Dihydroxybenzophenon; N,N-Di-(2-ethylhexyl)-4-aminobenzoesäureester mit 4-Hydroxydibenzoylmethan; 4-N,N-(2-Ethylhexyl)methyl-aminobenzoesäureester mit 4-Hydroxydibenzoylmethan; 4-N,N-(2-Ethylhexyl)methyl-aminobenzoesäureester von 2-Hydroxy-4-(2-hydroxyethoxy)benzophenon; 4-N,N-(2-Ethylhexyl)-methylaminobenzoesäureester von 4-(2-Hydroxyethoxy)dibenzoylmethan; N,N-Di-(2-ethylhexyl)-4-aminobenzoesäureester von 2-Hydroxy-4-(2-hydroxyethoxy)benzophenon und N,N-Di-(2-ethylhexyl)-4-aminobenzoesäureester von 4-(2-Hydroxyethoxy)dibenzoylmethan und Mischungen davon. Die genauen Mengen hängen von dem/den gewählten Sonnenschutzmittel(n) und dem gewünschten Lichtschutzfaktor (LSF) ab.

Konditionierungsmittel: Die erfindungsgemässen Zusammensetzungen können ein Konditionierungsmittel ausgewählt aus Feuchthaltemitteln, Feuchtigkeitsspendern oder Hautkonditionierungsmitteln enthalten. Diese Materialien schliessen Guanidin, Harnstoff, Glykolsäure und Glykolatsalze (z. B. Ammonium und quaternäres Alkylammonium), Salicylsäure; Milchsäure und Lactatsalze (z. B. Ammonium und quaternäres Alkylammonium); Aloe vera in beliebigen seiner vielen verschiedenen Formen (z. B. Aloe vera-Gel); Polyhydroxyalkohole wie Sorbitol, Mannitol, Xylitol, Erythritol, Glycerin, Hexantriol, Butantriol, Propylenglykol, Butylenglykol, Hexylenglykol und dergleichen; Polyethylenglykole; Zucker (z. B. Melibiose) und Stärken, Zucker- und Stärkederivate (z. B. alkoxylierte Glucose, Fructose, Glucosamin), Hyaluronsäure, Lactamidmonoethanolamin, Acetamidmonoethanolamin, Panthenol, Allantoin und Mischungen davon ein, sind jedoch nicht auf diese begrenzt. Ebenfalls brauchbar sind die propoxylierten Glycerine, die in US-A-4,976,953, erteilt am 11. Dezember 1990, von Orr et al. beschrieben worden sind.

Ebenfalls brauchbar sind verschiedene C₁- bis C₃₀-Monoester und Polyester von Zuckern und verwandte Materialien. Diese Ester sind von einer Zucker- oder Polyolgruppe und einer oder mehreren Carbonsäuregruppen abgeleitet.

Das Konditionierungsmittel ist vorzugsweise ausgewählt aus Harnstoff, Guanidin, Sucrosepolyester, Panthenol, Dexpanthenol, Allantoin und Kombinationen davon.

Dermatologisch annehmbare Träger: Die erfindungsgemässen topischen Zusammensetzungen enthalten auch einen dermatologisch annehmbaren Träger. Die Formulierung "dermatologisch annehmbarer Träger" bedeutet hier, dass der Träger zur topischen Anwendung auf das Horngewebe geeignet ist, gute ästhetische Eigenschaften hat, mit den Wirkstoffen der vorliegenden Erfindung und beliebigen anderen Komponenten verträglich ist und nicht zu ungünstigen Sicherheits- oder Toxizitätsbedenken führt.

Der Träger kann in vielen unterschiedlichen Formen vorliegen. Beispielsweise sind hier Emulsionsträger einschliesslich Öl-in-Wasser-, Wasser-in-Öl-, Wasser-in-Öl-in-Wasser- und Öl-in-Wasser-in-Silikonemulsionen brauchbar.
A) Wasser-in-Silikon-Emulsion
   Wasser-in-Silikon-Emulsionen enthalten eine kontinuierliche Silikonphase und eine dispergierte wässrige Phase.
B) Öl-in-Wasser-Emulsionen
   Andere bevorzugte topische Träger schliessen Öl-in-Wasser-Emulsionen mit einer kontinuierlichen wässrigen Phase und einer darin dispergierten hydrophoben, wasserunlöslichen Phase ("Ölphase") ein. Beispiele für geeignete Öl-in-Wasser-Emulsionsträger sind in US-A-5,073,371 von D. J. Turner et al., erteilt am 17. Dezember 1991, und US-A-5,073,372 von D. J. Turner et al., erteilt am 17. Dezember 1991, beschrieben.

### Beispiele:

Die folgenden Beispiele sollen die Erfindung erläutern ohne sie einzuschränken. Benutzte Abkürzungen im Text und in den Beispielen 1-7:
AcOH: Essigsäure
AK: Antikörper
Boc: tert.-Butyloxycarbonyl
BSA: Bovine Serum Albumine
Dab: 2,4-Diaminobuttersäure
Dap: 2,3-Diaminopropionsäure
DBU: 1,8-Diazabicyclo[5,4,0]undec-7-ene(1,5-5)
FCS: Foetal Calf Serum
TFA: Trifluoressigsäure
Gly: Glycin
HSe: Homoserin
ILe: Isoleucin
MEM: Minimal Essential Medium
NEAA: Non Essential Amino Acids
NLe: Norleucin
NVa: Norvalin
Orn: Ornithin
Palm: Palmitoyl
PBS: Phosphate buffered saline
Pe: Petrolether
RT: Raumtemperatur
tBu: tert.-Butyl
tBuGly: tert.-Butylglycin
TBTU: O-(Benzotriazol-1-yl)-N,N,N',N',-tetramethyluroniumtetrafluoroborat
TGFβ1: Transforming Growth Factor-β1

### Beispiel 1: Bestimmung der Stimulierung der Kollagensynthese Typ I in Fibroblasten-Zellkulturen durch Behandlung mit den erfindungsgemässen Tripeptidderivaten

Typ I Kollagen von in-vitro kultivierten Hautfibroblasten wurde mittels eines ELISA (Enzyme-Linked Immunosorbent Assay) detektiert. In Gegenwart der peptidischen Wirkstoffe wurde die Steigerung der Kollagenproduktion der Zellen mit dieser Methode quantifiziert.
Die humanen Hautfibroblasten wurden aus Vorhaut isoliert und im Kulturmedium gezüchtet.
Nach 72h Inkubationszeit mit den entsprechenden Peptiden (Wirkstoff), erfolgt die quantitative Bestimmung mit einem für Kollagen I spezifischen Antikörper.

### Material:

| Kulturmedium: | Testmedium: |
|---|---|
| - MEM | - MEM |
| - 10% FCS | - kein FCS |
| - 100IU/ml Penicillin | - 100IU/ml Penicillin |
| - 0.1mg/ml Streptomycin | - 0.1mg/ml Streptomycin |
| - 1mM NEAA | - 1mM NEAA |
| - 1mM Na-Pyruvat | - 1mM Na-Pyruvat |
| - 2mM L-Glutamin | - 2mM L-Glutamin |
| - 20mM Hepes Buffer | - 20mM Hepes Buffer |

| Waschbuffer: | Milchlösung: |
|---|---|
| - 0.05M Tris, pH 8.5 | - Waschbuffer |
| - 0.15M NaCl | - 5% Milchpulver |
| - 0.1 % BSA | |
| - 0.1 % Tween-20 | |

| AK-Verdünnungslösung: | Substratlösung: |
|---|---|
| - 50ml SuperBlock (37515; Pierce) | - 1 ImmunoPure® OPD Tablet (34006; Pierce) |
| - 450ml H₂O | - 9ml H₂O |
| - 0.05% Tween | - 1ml Stable Peroxide Substr. |
| | Buffer, 10x (34062; Pierce) |

| | |
|---|---|
| 1. AK (MAB1340; Chemicon) und 2. AK (31430; Socochim S.A.) werden mit AK-Verd.Lsg 1/500 verdünnt. | |

### Methode:

Die Fibroblasten werden mit einer Dichte von ca. 5000 Zellen/Well in 96well-plates für 3Tage bis zur Konfluenz im Kulturmedium inkubiert (37 °C / 5% CO₂. Das Medium wird gegen Testmedium mit drei unterschiedlichen Konzentrationen in triplicate an Testsubstanz ausgetauscht. Folgende Kontrollen werden auf jeder Platte mitgetestet:

| Negativkontrollen: | Positivkontrollen: |
|---|---|
| A) | A) |
| - mit Zellen | - mit Zellen |
| - ohne 1.AK; mit 2.AK | - mit 1. und 2. AK |

| B) | B) |
|---|---|
| - ohne Zellen | - mit Zellen |
| - mit 1. und 2. AK | - mit 1. und 2. AK |
| | - mit 10ng/ml TGF-β1 |

C) Für jedes Peptid wird ein well ohne Zellen getestet, um die unspezifische Bindung der beiden AK auszuschließen.

Die Platten werden für weitere 72 Stunden inkubiert. Nach Abschluss dieser Inkubationszeit wird das abgelagerte Kollagen I nach folgendem Protokoll detektiert und quantifiziert:
■ Medium verwerfen und mit 200µl/well PBS waschen
■ mit 100µl/well Methanol fixieren -> 15min/ RT/ Shaker 600rpm
■ Methanol verwerfen und mit 200µl/well Milchlösung blockieren -> 30min/ RT/ Shaker 600rpm
■ Milchlösung verwerfen und mit 100µl/well 1.AK-Verd. inkubieren -> 2h/ RT/ Shaker 600rpm
■ 1.AK-Verd. verwerfen und 3x mit 200µl/well Washbuffer waschen
■ mit 100µl/well 2.AK-Verd. inkubieren -> 3h/ RT/ Shaker 600rpm
■ 2.AK-Verd. verwerfen; 3x mit 200µl/well Washbuffer und 1x 100µl/well PBS waschen
■ add 100µl/well Substratlösung -> 20min/ RT/ Shaker 600rpm
■ mit 50µl/well H₂SO₄ (2M) Reaktion stoppen und bei 492nm messen.

**Tabelle 1: Kollagenstimulation im ELISA:**

| Nr | Substanz | Konz /[µmol/L] | %Stimulation |
|---|---|---|---|
| | Kontrolle ohne Wirkstoff | - | 0 |
| | Referenzverbindung A (Elaidoyl-Lys-Phe-Lys-OH *2AcOH) | 0.01 / 20.0 | 23 43 |
| 1 | Elaidoyl-Lys-Thr-Lys-OH *2AcOH | 0.01 | 36 |
| 2 | Elaidoyl-Lys-Val-Lys-OH *2AcOH | 0.01 | 30 |
| 3 | Palm-Lys-Thr-Lys-OH *2AcOH | 1.56 | 35 |
| 4 | Palm-Lys-Val-Lys-OH *2AcOH | 25.0 | 104 |
| 5 | Palm-Dap-Val-Lys-OH *2TFA | 50 | 35 |
| 6 | Palm-Dap-Val-Lys-OH *2TFA | 50 | 48 |
| 7 | Myristoyl-Lys-Val-Lys-OH *2TFA | 100 | 61 |
| 8 | Palm-Lys-Val-Orn-OH *2TFA | 25 | 31 |
| 9 | Palm-Lys-Ile-Lys-OH *2TFA | 25 | 38 |
| 10 | H₂₉C₁₄-NH-CO-Lys-Val-Lys-OH *2TFA | 50 | 63 |
| 11 | H₃₃C₁₆-NH-CO-Lys-Val-Lys-OH*2TFA | 50 | 41 |
| 12 | H₃₁C₁₈-NH-CO-Lys-Val-Lys-OH*2TFA | 50 | 25 |
| 13 | Palm-Lys-Val-Dap-OH *2TFA | 100 | 154 |
| 14 | Palm-Lys-Val-Dab-OH *2TFA | 25 | 60 |
| 15 | Palm-Arg-Val-Arg-OH *2TFA | 50 | 49 |

### Beispiel 2: Formulierung einer Salbe

Prozedur: Die Inhaltstoffe 1-5 (A) werden auf 70°C erhitzt. Die Inhaltstoffe 6-7 (B) werden auf 75°C erhitzt. Unter Rühren wird B zu A gegeben, auf 50°C gekühlt, homogenisiert und auf 30°C abgekühlt. Dann werden die Inhaltstoffe 8-9 (C) und der Inhaltsstoff 10 (D) nacheinander hinzugegeben und kalt gerührt.

| Nummer | Inhaltsstoff | | % w/w |
|---|---|---|---|
| 1 | (A) | Tego Care 450 | 3.00 |
| 2 | | Cetearylalkohol | 2.25 |
| 3 | | Glycerylstearat | 2.25 |
| 4 | | Cetiol 868 | 10.00 |
| 5 | | Squalane | 5.00 |
| 6 | (B) | Deionisiertes Wasser | 66.995 |
| 7 | | Natriumhyaluronat | 5.00 |
| 8 | (C) | Glycerin | 5.00 |
| 9 | | Phenonip | 0.5 |
| 10 | (D) | Palm-Lys-Val-Lys-OH | 0.005 |

### Beispiel 3: Formulierung eines Gels

Prozedur: Die Inhaltstoffe 2-6 (A) werden nacheinander in deionisiertem Wasser gelöst. Mit Inhaltstoff 7 (B) wird auf pH 6.0 gestellt. Dann wird Inhaltstoff 8 (C) hinzugegeben.

| Nummer | Inhaltsstoff | | % w/w |
|---|---|---|---|
| 1 | (A) | Deionisiertes Wasser | 92.095 |
| 2 | | 1,3-Butandiol | 5.00 |
| 3 | | Phenonip | 0.50 |
| 4 | | Abil B 8843 | 1.50 |
| 5 | | Carboxymethyl Cellulose | 0.15 |
| 6 | | Carbopol Ultrez 10 | 0.75 |
| 7 | (B) | NaOH | |
| 8 | (C) | Palm-Lys-Val-Lys-OH | 0.005 |

### Beispiele 4-8:

In den nachfolgenden Ausführungsbeispielen 4-8 wird die Herstellung von erfindungsgemässen Verbindungen der Formel (I) und von Salzen solcher Verbindungen beschrieben. Die Analyse der gemäss den Beispielen erhaltenen Eluate und Produkte wurde mit Proton-NMR, HPLC-Elektrospray-MS oder Elementaranalyse ausgeführt. Die Verbindungen können nach den im Folgenden beschriebenen an sich bekannten Verfahren (allgemeinen Vorschriften von M. Bodanszky "The Practice of Peptide Synthesis" Springer Verlag, 2^{nd} Edition 1994) hergestellt werden. Entsprechend wird die Aminosäure, beispielsweise Lysin, in einer Festphasensynthese am carboxyterminalen Ende an ein Harz angeknüpft, wobei deren Aminogruppe durch eine Schutzgruppe, z.B. durch die Fmoc-Schutzgruppe, geschützt wird. Die Seitenkette wird z.B. mit Boc oder t-Butyl geschützt. Die Schutzgruppen werden nach Bedarf selektiv abgespalten, um die weiteren Aminosäure Derivate mit den in der Peptidsynthese üblichen Reagenzien anzuknüpfen bis die gewünschte Sequenz vollständig aufgebaut ist. Das Peptid wird dann am carboxyterminalen Ende vom Harz abgespalten und das rohe Peptid durch Eintropfen in ein geeignetes Lösungsmittelgemisch ausgefällt. Das Gemisch wird über HPLC gereinigt, ggf. in die Gegenionen ausgetauscht und die Substanz lyophylisiert.

### Beispiel 4: Elaidoyl-Lys-Thr-Lys-OH *2TFA

An 1.00 g (0.78 mmol) H-Lys(Boc)-2-Chlortritylharz wird mit Fmoc-Thr(tBu)-OH, Fmoc-Lys(Boc)-OH und Elaidoyl-OSu das geschützte Peptid am Harz aufgebaut. Das Harz wird für 30 min mit 8 ml 95% TFA behandelt, die Lösung in 100 ml Et₂O eingetropft. Der Niederschlag wird abgesaugt, nachgewaschen und nach dem Trocknen über präp. HPLC gereinigt und lyophylisiert.
Ausbeute: 73mg (0.097mmol, 12%)

### Beispiel 5: H-Lys-Thr-Lys-OH *3TFA

An 3.00 g (2.58 mmol) H-Lys(Boc)-2-Chlortritylharz wird mit Fmoc-Thr(tBu)-OH und Z-Lys(Z)-OH das geschützte Peptid am Harz aufgebaut. Das Harz wird für 30 min mit 20 ml 95% TFA behandelt, die Lösung in 400 ml ^{t}BuOMe:PE=1:1 eingetropft. Der Niederschlag wird abgesaugt, nachgewaschen und nach dem Trocknen über präp. HPLC gereinigt.
Das teilgeschützte Peptid wird in 50ml Dioxan:Wasser = 4:6 gelöst, mit 200mg Pd/C und 3 Eq TFA versetzt und 5h unter H₂-Atmosphäre reduziert. Es wird über Celite filtriert, einrotiert, über präp. HPLC gereinigt und lyophylisiert.

### Beispiel 6: Palm-Lys-Val-Lys-OH *2AcOH

An 1.00 g (0.80 mmol) H-Lys(Boc)-2-Chlortritylharz wird mit Fmoc-Val-OH, Fmoc-Lys(Boc)-OH und Palm-OSu das geschützte Peptid am Harz aufgebaut. Das Harz wird für 30 min mit 8 ml 95% TFA behandelt, die Lösung in 100 ml Et₂O eingetropft. Der Niederschlag wird abgesaugt, nachgewaschen und nach dem Trocknen über präp. HPLC gereinigt. Die Substanz wird in 30ml Dioxan: Wasser = 4:6 gelöst, mit 2.0g BioRad Harz (Acetatform) über Nacht behandelt, filtriert, einrotiert, und lyophylisiert.
Ausbeute 110mg (0.15 mmol, 19%)

### Beispiel 7: H-Lys-Val-Lys-NH-Cetyl *3TFA

An 13.5 g (10.8mmol) H-Lys(Boc)-2-Chlortritylharz wird mit Fmoc-Val-OH und Boc-Lys(Boc)-OH das geschützte Peptid am Harz aufgebaut. Das Harz wird für 3*10 min mit 80 ml 1% TFA in Methylenchlorid behandelt, die Lösung mit Pyridin:Methanollösung neutralisiert und über präp HPLC gereinigt.
Ausbeute: Boc-Lys(Boc)-Val-Lys(Boc)-OH 4.66g (6.915 mmol, 64%)
Zu 150 mg (0.223 mmol) Boc-Lys(Boc)-Val-Lys(Boc)-OH in 5ml DMF gibt man 84 □l (0.441mmol) DIPEA, 78.5 mg (0.245mmol) TBTU und 59.1mg (0.245mmol) Cetylamin. Nach 30 Min wird die Reaktionslösung wässrig exrahiert, Der Rückstand aus der organischen Phase 30 min mit 95% TFA behandelt und über Präp HPLC gereinigt.
Ausbeute: H-Lys-Val-Lys-NH-Cetyl *3TFA 48.2mg (0.051 mmol, 23%)

### Beispiel 8: H-Lys-Val-Lys-O-Oktyl *3TFA

10 ml Oktanol werden auf -10°C gekühlt und vorsichtig 75 ml SOC12 (1.03mmol) zugegeben. Nach 10 min gibt man 150 mg (0.223 mmol) Boc-Lys(Boc)-Val-Lys(Boc)-OH zu ind lässt 3 Tage rühren. Reinigung über Präp HPLC ergibt das Produkt. Ausbeute: H-Lys-Val-Lys-O-Oktyl *3TFA 165.6mg (0.200 mmol, 90%)

## Patentansprüche

1. Tripeptidderivate der allgemeinen Formel I 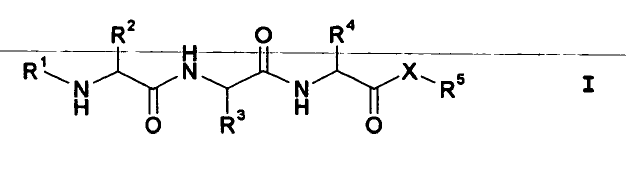 worin
R¹ = H, -C(O)-R⁶, -SO₂-R⁶ oder -C(O)-XR⁶
R² und R⁴ = unabhängig von einander (CH₂)ₙ-NH₂ oder (CH₂)₃-NHC(NH)NH₂,
n = 1-4,
R³ = gegebenenfalls mit Hydroxy substituiertes, lineares oder verzweigtes C₁-C₄-Alkyl,
R⁵ und R⁶ = unabhängig von einander Wasserstoff, gegebenenfalls substituiertes (C₁-C₂₄)-Alkyl, gegebenenfalls substituiertes C₂-C₂₄-Alkenyl, gegebenenfalls substituiertes Phenyl, gegebenenfalls substituiertes Phenyl-C₁-C₄-alkyl oder 9-Fluorenylmethyl, X = Sauerstoff (-O-) oder -NH-; oder
XR⁵ mit X = O auch die Ester von α-Tocopherol, Tocotrienol oder Retinol bedeuten,
mit der Massgabe, dass weder gleichzeitig R¹ und R⁵ Wasserstoff und X Sauerstoff bedeuten noch gleichzeitig R¹ β-Methoxyacryl, R² und R⁴ (CH₂)₄-NH₂, X Sauerstoff und R⁵ Methyl bedeuten.

2. Verbindungen nach Anspruch 1, **dadurch gekennzeichnet, dass** R¹ = -C(O)-R⁶ bedeutet.

3. Verbindungen nach Anspruch 1, **dadurch gekennzeichnet, dass** R² und R⁴ (CH₂)ₙ-NH₂ bedeuten.

4. Verbindungen nach Anspruch 1, **dadurch gekennzeichnet, dass** R⁵ Wasserstoff bedeutet.

5. Verbindungen nach einem der Ansprüche 1-4, **dadurch gekennzeichnet, dass** die Verbindungen der Formel I mit Säuren ein- oder mehrwertige, einheitliche oder gemischte Salze bilden, vorzugsweise mit anorganischen Säuren, oder mit geeigneten organischen alphatischen gesättigten oder ungesättigten Carbonsäuren, oder mit aromatischen Carbonsäuren, oder mit aroma-tisch-aliphatischen Carbonsäuren, oder mit heteroaromatischen Carbonsäuren, oder mit aliphatischen oder aromatischen Sulfon-säuren, vorzugsweise mit Essigsäure, Trifluoressigsäure und/oder Milchsäure.

6. Verbindungen nach einem der Ansprüche 1-5, **dadurch gekennzeichnet, dass** sie als isomerenreine Formen oder Gemische verschiedener Isomeren, und als Gemische von Rotameren vorliegen.

7. Verbindungen nach einem der Ansprüche 1-6, **dadurch gekennzeichnet, dass**
R¹ = -C(O)-R⁶,
R² und R⁴ = unabhängig von einander (CH₂)ₙ-NH₂
n = 1-4,
R³ = gegebenenfalls mit Hydroxy substituiertes, lineares
oder verzweigtes C₁-C₄-Alkyl,
R⁵ = Wasserstoff, gegebenenfalls substituiertes (C₁-C₂₄)-Alkyl oder gegebenenfalls substituiertes C₂-C₂₄-Alkenyl,
R⁶ = gegebenenfalls substituiertes (C₁-C₂₄)-Alkyl oder gegebenenfalls substituiertes C₂-C₂₄-Alkenyl, und
X = Sauerstoff (-O-) oder -NH-; oder
XR⁵ mit X = O auch die Ester von α-Tocopherol, Tocotrienol
oder Retinol bedeuten.

8. Verbindungen der Formeln
Elaidoyl-Lys-Val-Lys-OH
Elaidoyl-Lys-Thr-Lys-OH
Palm-Lys-Thr-Lys-OH
Palm-Lys-Val-Lys-OH
Palm-Orn-Val-Lys-OH
Palm-Orn-Val-Dab-OH
Palm-Orn-Val-Dap-OH
Palm-Dab-Val-Lys-OH
Palm-Dab-Val-Dab-OH
Palm-Dab-Val-Dap-OH
Palm-Dap-Val-Lys-OH
Palm-Dap-Val-Dab-OH
Palm-Dap-Val-Dap-OH
Palm-Arg-Val-Lys-OH
Palm-Arg-Val-Dab-OH
Palm-Arg-Val-Dap-OH
Palm-Lys-Val-Lys-OH
Palm-Lys-Val-Orn-OH
Palm-Lys-Val-Dab-OH
Palm-Lys-Val-Dap-OH
Palm-Lys-Val-Arg-OH
Palm-Lys-Leu-Lys-OH
Palm-Lys-ILe-Lys-OH
Palm-Lys-ILe-Dab-OH
Palm-Lys-NVa-Dab-OH
Palm-Lys-tBuGly-Dab-OH
Palm-Lys-Leu-Dab-OH
Palm-Lys-ILe-Dap-OH
Palm-Lys-NVa-Dap-OH
Palm-Lys-tBuGly-Dap-OH
Palm-Lys-Leu-Dap-OH
Palm-Lys-NLe-Lys-OH
Palm-Lys-Ala-Lys-OH
Palm-Lys-Ser-Lys-OH
Palm-Lys-HSe-Lys-OH
Palm-Arg-Val-Arg-OH
Pentadecanoyl-Lys-Val-Dab-OH
Pentadecanoyl-Lys-Val-Dap-OH
Heptadecanoyl-Lys-Val-Dab-OH
Heptadecanoyl-Lys-Val-Dap-OH
Myristoyl-Lys-Val-Lys-OH
Myristoyl-Lys-Val-Dab-OH
Myristoyl-Lys-Val-Dap-OH
Lauroyl-Lys-Val-Lys-OH
Caprinoyl-Lys-Val-Lys-OH
Stearoyl-Lys-Val-Lys-OH
Stearoyl-Lys-Val-Dab-OH
Stearoyl-Lys-Val-Dap-OH
Oleolyl-Lys-Val-Lys-OH
Palm-Lys-Val-Dab-OMe
Palm-Lys-Val-Dab-OOctyl
Palm-Lys-Val-Dab-OCetyl
Palm-Lys-Val-Dab-NH₂
Palm-Lys-Val-Dab-NHBu
Palm-Lys-Val-Dab-NHOctyl
Palm-Lys-Val-Dab-NHCetyl
Palm-Lys-Val-Dap-OMe
Palm-Lys-Val-Dap-OOctyl
Palm-Lys-Val-Dap-OCetyl
Palm-Lys-Val-Dap-NH₂
Palm-Lys-Val-Dap-NHBu
Palm-Lys-Val-Dap-NHOctyl
Palm-Lys-Val-Dap-NHCetyl
C₁₄H₂₉-NH-CO-Lys-Val-Lys-OH
C₁₄H₂₉-NH-CO-Dab-Val-Dab-OH
C₁₄H₂₉-NH-CO-Lys-Ile-Dab-OH
C₁₄H₂₉-NH-CO-Lys-Val-Dap-OH
C₁₄H₂₉-NH-CO-Arg-Val-Arg-OH
C₁₄H₂₉-NH-CO-Dab-Val-Dap-OH
C₁₄H₂₉-NH-CO-Lys-Ile-Dap-OH
C₁₄H₂₉-NH-CO-Lys-Val-Dab-OH
C₁₄H₂₉-NH-CO-Dap-Val-Dap-OH
C₁₆H₃₃-NH-CO-Lys-Val-Lys-OH
C₁₈H₃₇-NH-CO-Lys-Val-Lys-OH
Boc-Lys-Val-Lys-OH
Ac-Lys-Val-Lys-OH
Z-Lys-Val-Lys-OH
Fmoc-Lys-Val-Lys-OH
C₈H₁₇-SO₂-Lys-Val-Lys-OH
C₁₆H₃₃-SO₂-Lys-Val-Lys-OH
H-Lys-Val-Lys-NH₂
H-Lys-Val-Lys-OOktyl
H-Lys-Val-Lys-OCetyl
H-Lys-Val-Lys-ORetinyl
H-Lys-Val-Lys-OTocopheryl
H-Lys-Val-Lys-OBn
H-Lys-Val-Lys-NH-Cetyl
H-Lys-Val-Lys-NH-Oktyl
H-Lys-Val-Lys-NH-Bn
und ihre Säureadditionssalze.

9. Verfahren zur Herstellung der Verbindungen nach einem der Ansprüche 1-8, **dadurch gekennzeichnet, dass** man unter Verwendung von an sich in der Peptidchemie bekannten Methoden die Verbindung der Formel I vollständig aufbaut, gegebenenfalls die verbleibende(n) Schutzgruppe(n) abspaltet und gegebenenfalls eine freie Aminogruppe acyliert oder sulfonyliert und/oder die erhaltene Verbindung in ein Säureadditionssalz und /oder ein erhaltenes Säureadditionssalz in die entsprechende konjugate Base oder in ein anderes Salz überführt.

10. Verwendung der Verbindungen nach einem der Ansprüche 1-8 als kosmetischen Wirkstoff, insbesondere für die Erhöhung der Produktion von Collagen und/oder Elastin in der menschlichen Haut und/oder zur Verzögerung oder Behandlung der Hautalterung.

11. Verwendung der Verbindungen nach einem der Ansprüche 1-8 zur Herstellung von kosmetisch wirksamen Zusammensetzungen, insbesondere als Hautpflegemittel, insbesondere zur Verhinderung der Bildung und der Verschlimmerung der Falten und gegen alle Folgen der Hautalterung natürlicher (endogener) oder beschleunigter (exogener) Art.

12. Kosmetisch wirksame Zusammensetzung, welche mindestens eine Verbindung nach einem der Ansprüche 1-8 enthält, vorzugsweise in einer Menge im Bereich zwischen 0.5 ppm und 1'000 ppm (w/w), vorzugsweise zwischen 1 ppm und 100 ppm (w/w), berechnet auf das Gewicht der erfindungsgemässen Verbindung und des Trägermaterials bzw. der Trägermaterialien.

13. Kosmetisch wirksame Zusammensetzung, **dadurch gekennzeichnet, dass** diese enthält:
a) mindestens ein Tripeptidderivat der allgemeinen Formel (I) nach einem der Ansprüche 1-8, und
b) eine sichere und wirksame Menge von mindestens einem zusätzlichen Hautpflegewirkstoff ausgewählt aus der Gruppe bestehend aus Wirkstoffen für die Abschuppung, Anti-Aknewirkstoffen, Vitamin B3-Verbindungen, Retinoiden, Di-, Tri-, Tetra- und Pentapeptiden und Derivaten davon, Hydroxysäuren,Radikalfänger, entzündungshemmenden Mitteln, Hautbräunungswirkstoffen, Hautaufhellungsmitteln, Anti-Cellulitismitteln, Flavonoiden, antimikrobiellen Wirkstoffen, Hautheilungsmitteln, antimykotischen Wirkstoffen, Sonnenschutzwirkstoffen, Farnesol, Phytantriol, Allantoin, Glucosamin und Mischungen davon.

14. Hautpflegezusammensetzung nach Anspruch 13, welche einen dermatologisch annehmbaren Träger enthält.

15. Zusammensetzung nach einem der Ansprüche 12-14, **dadurch gekennzeichnet, dass** diese in Form einer Lösung, einer Dispersion, einer Emulsion oder eingekapselt in Trägern, vorzugsweise in Makro-, Mikro- oder Nanokapseln, in Liposomen oder Chylomikronen, oder eingeschlossen in Makro-, Mikro- oder Nanoteilchen oder in Mikroschwämme oder absorbiert auf pulverförmigen organischen Polymeren, Talk, Bentonit und anderen mineralischen Trägern, vorliegt.

16. Zusammensetzung nach einem der Ansprüche 12-14, **dadurch gekennzeichnet, dass** diese in Form einer Emulsion, Milch, Lotio, Salbe, eines gelierenden und viskosen, spannungsaktiven und emulgierenden Polymeren, einer Pommade, eines Shampoos, einer Seife, eines Gels, Puders, Sticks oder Stifts, Sprays, Körperöls, einer Gesichtsmaske oder eines Pflasters zur transdermalen Applikation, vorliegt.

17. Zusammensetzung nach einem der Ansprüche 12-16 als kosmetisch wirksames Mittel, insbesondere für die Erhöhung der Produktion von Collagen und Elastin in der menschlichen Haut, als Hautpflegemittel, insbesondere zur Verhinderung der Bildung und der Verschlimmerung der Falten und gegen alle Folgen der Hautalterung natürlicher (endogener) oder beschleunigter (exogener) Art (Heliodermie, Verschmutzung).

18. Kosmetisches Verfahren zur Erhöhung der Produktion von Collagen und/oder Elastin in der menschlichen Haut und/oder zur Verzögerung oder Behandlung der Hautalterung, **dadurch gekennzeichnet, dass** man eine Verbindung nach einem der Ansprüche 1-8 auf die Haut appliziert.

## Claims

1. Tripeptide derivatives of general formula I 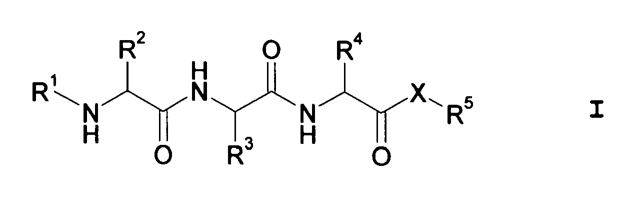 wherein
R¹ represents H, -C(O)-R⁶, -SO₂-R⁶ or -C(O)-XR⁶
R² and R⁴, independent of one another, represent (CH₂)ₙ-NH₂ or (CH₂)₃-NHC(NH)NH₂,
n equals 1-4,
R³ represents linear or branched C₁-C₄-alkyl that is optionally substituted by hydroxy,
R⁵ and R⁶, independent of one another, represent hydrogen, optionally substituted (C₁-C₂₄)-alkyl, optionally substituted C₂-C₂₄-alkenyl, optionally substituted phenyl, optionally substituted phenyl-C₁-C₄-alkyl or 9-fluorenylmethyl,
X represents oxygen (-O-) or -NH-; or
XR⁵ with X = O also represents the esters of α-tocopherol, tocotrienol or retinol, with the provision that R¹ and R⁵ do neither represent hydrogen and X oxygen simultaneously, nor that R¹ represents β-methoxyacryl, R² and R⁴ (CH₂)₄-NH₂, X oxygen and R⁵ methyl simultaneously.

2. Compounds according to claim 1, thereby **characterized that** R¹ represents - C(O)-R⁶.

3. Compounds according to claim 1, thereby **characterized that** R² and R⁴ represent (CH₂)ₙ-NH₂.

4. Compounds according to claim 1, thereby **characterized that** R⁵ represents hydrogen.

5. Compounds according to one of claims 1-4, thereby **characterized that** the compounds of formula I together with acids can form mono- or polyvalent, homogeneous or mixed salts, preferably with inorganic acids, or with appropriate organic aliphatic saturated or unsaturated carboxylic acids, or with aromatic carboxylic acids, or with aromatic-aliphatic carboxylic acids, or with heteroaromatic carboxylic acids, or with aliphatic or aromatic sulfonic acids, preferably with acetic acid, trifluoroacetic acid and/or lactic acid.

6. Compounds according to one of claims 1-5, thereby **characterized that** they are present as pure isomers or mixtures of different isomers, and as mixtures of rotamers.

7. Compounds according to one of claims 1-6, thereby **characterized that**
R¹ represents -C(O)-R⁶,
R² and R⁴, independent of one another, represent (CH₂)ₙ-NH₂
n equals 1-4,
R³ represents linear or branched C₁-C₄-alkyl optionally substituted by hydroxy,
R⁵ represents hydrogen, optionally substituted (C₁-C₂₄)-alkyl or optionally substituted C₂-C₂₄-alkenyl,
R⁶ represents optionally substituted (C₁-C₂₄)-alkyl or optionally substituted C₂-C₂₄-alkenyl, and
X represents oxygen (-O-) or -NH-; or
XR⁵ with X = O also represents the esters of α-tocopherol, tocotrienol or retinol.

8. Compounds of formulas
Elaidoyl-Lys-Val-Lys-OH
Elaidoyl-Lys-Thr-Lys-OH
Palm-Lys-Thr-Lys-OH
Palm-Lys-Val-Lys-OH
Palm-Orn-Val-Lys-OH
Palm-Orn-Val-Dab-OH
Palm-Orn-Val-Dap-OH
Palm-Dab-Val-Lys-OH
Palm-Dab-Val-Dab-OH
Palm-Dab-Val-Dap-OH
Palm-Dap-Val-Lys-OH
Palm-Dap-Val-Dab-OH
Palm-Dap-Val-Dap-OH
Palm-Arg-Val-Lys-OH
Palm-Arg-Val-Dab-OH
Palm-Arg-Val-Dap-OH
Palm-Lys-Val-Lys-OH
Palm-Lys-Val-Orn-OH
Palm-Lys-Val-Dab-OH
Palm-Lys-Val-Dap-OH
Palm-Lys-Val-Arg-OH
Palm-Lys-Leu-Lys-OH
Palm-Lys-Ile-Lys-OH
Palm-Lys-Ile-Dab-OH
Palm-Lys-Nva-Dab-OH
Palm-Lys-tBuGly-Dab-OH
Palm-Lys-Leu-Dab-OH
Palm-Lys-Ile-Dap-OH
Palm-Lys-Nva-Dap-OH
Palm-Lys-tBuGly-Dap-OH
Palm-Lys-Leu-Dap-OH
Palm-Lys-Nle-Lys-OH
Palm-Lys-Ala-Lys-OH
Palm-Lys-Ser-Lys-OH
Palm-Lys-Hse-Lys-OH
Palm-Arg-Val-Arg-OH
Pentadecanoyl-Lys-Val-Dab-OH
Pentadecanoyl-Lys-Val-Dap-OH
Heptadecanoyl-Lys-Val-Dab-OH
Heptadecanoyl-Lys-Val-Dap-OH
Myristoyl-Lys-Val-Lys-OH
Myristoyl-Lys-Val-Dab-OH
Myristoyl-Lys-Val-Dap-OH
Lauroyl-Lys-Val-Lys-OH
Caprinoyl-Lys-Val-Lys-OH
Stearoyl-Lys-Val-Lys-OH
Stearoyl-Lys-Val-Dab-OH
Stearoyl-Lys-Val-Dap-OH
Oleolyl-Lys-Val-Lys-OH
Palm-Lys-Val-Dab-OMe
Palm-Lys-Val-Dab-OOctyl
Palm-Lys-Val-Dab-OCetyl
Palm-Lys-Val-Dab-NH₂
Palm-Lys-Val-Dab-NHBu
Palm-Lys-Val-Dab-NHOctyl
Palm-Lys-Val-Dab-NHCetyl
Palm-Lys-Val-Dap-OMe
Palm-Lys-Val-Dap-OOctyl
Palm-Lys-Val-Dap-OCetyl
Palm-Lys-Val-Dap-NH₂
Palm-Lys-Val-Dap-NHBu
Palm-Lys-Val-Dap-NHOctyl
Palm-Lys-Val-Dap-NHCetyl
C₁₄H₂₉-NH-CO-Lys-Val-Lys-OH
C₁₄H₂₉-NH-CO-Dab-Val-Dab-OH
C₁₄H₂₉-NH-CO-Lys-Ile-Dab-OH
C₁₄H₂₉-NH-CO-Lys-Val-Dap-OH
C₁₄H₂₉-NH-CO-Arg-Val-Arg-OH
C₁₄H₂₉-NH-CO-Dab-Val-Dap-OH
C₁₄H₂₉-NH-CO-Lys-Ile-Dap-OH
C₁₄H₂₉-NH-CO-Lys-Val-Dab-OH
C₁₄H₂₉-NH-CO-Dap-Val-Dap-OH
C₁₆H₃₃-NH-CO-Lys-Val-Lys-OH
C₁₈H₃₇-NH-CO-Lys-Val-Lys-OH
Boc-Lys-Val-Lys-OH
Ac-Lys-Val-Lys-OH
Z-Lys-Val-Lys-OH
Fmoc-Lys-Val-Lys-OH
C₈H₁₇-SO₂-Lys-Val-Lys-OH
C₁₆H₃₃-SO₂-Lys-Val-Lys-OH
H-Lys-Val-Lys-NH₂
H-Lys-Val-Lys-OOctyl
H-Lys-Val-Lys-OCetyl
H-Lys-Val-Lys-ORetinyl
H-Lys-Val-Lys-OTocopheryl
H-Lys-Val-Lys-OBn
H-Lys-Val-Lys-NH-Cetyl
H-Lys-Val-Lys-NH-Octyl
H-Lys-Val-Lys-NH-Bn
and the acid addition salts thereof.

9. A method for synthesizing the compounds according to one of claims 1-8, thereby **characterized that** by applying methods known per se in peptide chemistry, it comprises fully assembling the compound of formula I, optionally splitting off the remaining protective group(s) and optionally acylating or sulfonylating a free amino group and/or converting the obtained compound into an acid addition salt and/or an obtained acid addition salt into the corresponding conjugate base or into another salt.

10. Use of the compounds according to one of claims 1-8 as a cosmetic active, in particular for increasing the production of collagen and/or elastin in human skin and/or for delaying or treating skin aging.

11. Use of the compounds according to one of claims 1-8 for the preparation of cosmetically active compositions, in particular as skin care products, in particular to prevent the formation and aggravation of wrinkles and against all consequences of natural (endogenous) or accelerated (exogenous) skin aging.

12. A cosmetically active composition comprising at least one compound according to one of claims 1-8, preferably in a quantity ranging between 0.5 ppm and 1,000 ppm (w/w), preferably between 1 ppm and 100 ppm (w/w), calculated on the weight of the compound of the present invention and of the carrier(s).

13. A cosmetically active composition thereby **characterized that** it contains
a) at least one tripeptide derivative of general formula (I) according to one of claims 1-8, and
b) a safe and efficient quantity of at least one additional skin care active selected from the group comprising desquamation actives, anti-acne agents, vitamin B3 compounds, retinoids, di-, tri-, tetra- and pentapeptides and derivatives thereof, hydroxy acids, radical scavengers, anti-inflammatory agents, skin-tanning actives, skin-whitening agents, anti-cellulite agents, flavonoids, antimicrobial actives, skin-healing agents, antifungal actives, sunscreens, farnesol, phytantriol, allantoin, glucosamine and mixtures thereof.

14. A skin care composition according to claim 13, that comprises a dermatologically acceptable carrier.

15. A composition according to one of claims 12-14, thereby **characterized that** it can be in the form of a solution, a dispersion, an emulsion or encapsulated in carriers, preferably in macro-, micro- or nanocapsules, in liposomes or chylomicrons, or enclosed in macro-, micro- or nanoparticles or in microsponges or absorbed on powdered organic polymers, talc, bentonite and other mineral carriers.

16. A composition according to one of claims 12-14, thereby **characterized that** it can be in the form of an emulsion, a milk, a lotion, an ointment, a gel-forming and viscous, surfactant and emulsifying polymer, a pomade, a shampoo, a soap, a gel, a powder, a stick or pencil, a spray, a body oil, a face mask or a plaster for transdermal application.

17. A composition according to one of claims 12-16 as a cosmetically active agent, in particular for increasing the production of collagen and elastin in human skin, as a skin care product, in particular to prevent the formation and aggravation of wrinkles and against all consequences of natural (endogenous) or accelerated (exogenous) skin aging.

18. A cosmetic method for increasing the production of collagen and/or elastin in human skin and/or for delaying or treating skin aging, thereby **characterized that** a compound according to one of claims 1-8 is applied onto the skin.

## Revendications

1. Dérivés tripeptidiques de formule générale 1 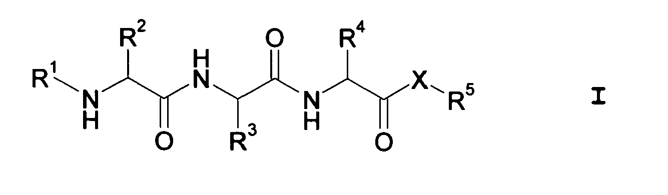 où
R¹ représente H, -C(O)-R⁶, -SO₂-R⁶ ou -C(O)-XR⁶
R² et R⁴, indépendamment l'un de l'autre, représentent (CH₂)ₙ-NH₂ ou (CH₂)₃-NHC(NH)NH₂,
n est égal à 1-4,
R³ représente C₁-C₄-alkyle linéaire ou ramifié, éventuellement substitué par hydroxy,
R⁵ et R⁶, indépendamment l'un de l'autre, représentent l'hydrogène, (C₁-C₂₄)-alkyle éventuellement substitué, C₂-C₂₄-alkényle éventuellement substitué, phényle éventuellement substitué, phényl-C₁-C₄-alkyle éventuellement substitué ou 9-fluorénylméthyle,
X représente l'oxygène (-O-) ou -NH-; ou
XR⁵ avec X = O représente également les esters de α-tocophérol, tocotriénol ou rétinol,
à la condition que R¹ et R⁵ ne représentent pas l'hydrogène et X l'oxygène simultanément, ni que R¹ représente β-méthoxy-acryle, R² et R⁴ (CH₂)₄-NH₂, X l'oxygène et R⁵ méthyle simultanément.

2. Composés selon la revendication 1, **caractérisés en ce que** R¹ représente - C(O)-R⁶.

3. Composés selon la revendication 1, **caractérisés en ce que** R² et R⁴ représentent (CH₂)ₙ-NH₂.

4. Composés selon la revendication 1, **caractérisés en ce que** R⁵ représente l'hydrogène.

5. Composés selon l'une des revendications 1-4, **caractérisés en ce que** les composés de formule I associés à des acides peuvent former des sels mono- ou polyvalents, homogènes ou mixtes, de préférence avec des acides minéraux, ou avec des acides carboxyliques organiques aliphatiques saturés ou insaturés adéquats, ou avec des acides carboxyliques aromatiques, ou avec des acides carboxyliques aromatico-aliphatiques, ou avec des acides carboxyliques hétéroaromatiques, ou avec des acides sulfoniques aliphatiques ou aromatiques, de préférence avec de l'acide acétique, de l'acide trifluoroacétique et/ou de l'acide lactique.

6. Composés selon l'une des revendications 1-5, **caractérisés en ce qu'**ils se présentent sous la forme d'isomères purs ou de mélanges de différents isomères, et sous forme de mélanges de rotamères.

7. Composés selon l'une des revendications 1-6, **caractérisés en ce que**
R¹ représente -C(O)-R⁶,
R² et R⁴, indépendamment l'un de l'autre, représentent (CH₂)ₙ-NH₂
n est égal à 1-4,
R³ représente C₁-C₄-alkyle linéaire ou ramifié, éventuellement substitué par hydroxy,
R⁵ représente l'hydrogène, (C₁-C₂₄)-alkyle éventuellement substitué ou C₂-C₂₄-alkényle éventuellement substitué,
R⁶ représente (C₁-C₂₄)-alkyle éventuellement substitué ou C₂-C₂₄-alkényle éventuellement substitué, et
X représente l'oxygène (-O-) ou -NH-; ou
XR⁵ avec X = O représente également les esters de α-tocophérol, tocotriénol ou rétinol.

8. Composés de formules
Elaidoyl-Lys-Val-Lys-OH
Elaidoyl-Lys-Thr-Lys-OH
Palm-Lys-Thr-Lys-OH
Palm-Lys-Val-Lys-OH
Palm-Orn-Val-Lys-OH
Palm-Orn-Val-Dab-OH
Palm-Orn-Val-Dap-OH
Palm-Dab-Val-Lys-OH
Palm-Dab-Val-Dab-OH
Palm-Dab-Val-Dap-OH
Palm-Dap-Val-Lys-OH
Palm-Dap-Val-Dab-OH
Palm-Dap-Val-Dap-OH
Palm-Arg-Val-Lys-OH
Palm-Arg-Val-Dab-OH
Palm-Arg-Val-Dap-OH
Palm-Lys-Val-Lys-OH
Palm-Lys-Val-Orn-OH
Palm-Lys-Val-Dab-OH
Palm-Lys-Val-Dap-OH
Palm-Lys-Val-Arg-OH
Palm-Lys-Leu-Lys-OH
Palm-Lys-Ile-Lys-OH
Palm-Lys-Ile-Dab-OH
Palm-Lys-Nva-Dab-OH
Palm-Lys-tBuGly-Dab-OH
Palm-Lys-Leu-Dab-OH
Palm-Lys-Ile-Dap-OH
Palm-Lys-Nva-Dap-OH
Palm-Lys-tBuGly-Dap-OH
Palm-Lys-Leu-Dap-OH
Palm-Lys-Nle-Lys-OH
Palm-Lys-Ala-Lys-OH
Palm-Lys-Ser-Lys-OH
Palm-Lys-Hse-Lys-OH
Palm-Arg-Val-Arg-OH
Pentadécanoyl-Lys-Val-Dab-OH
Pentadécanoyl-Lys-Val-Dap-OH
Heptadécanoyl-Lys-Val-Dab-OH
Heptadécanoyl-Lys-Val-Dap-OH
Myristoyl-Lys-Val-Lys-OH
Myristoyl-Lys-Val-Dab-OH
Myristoyl-Lys-Val-Dap-OH
Lauroyl-Lys-Val-Lys-OH
Caprinoyl-Lys-Val-Lys-OH
Stéaroyl-Lys-Val-Lys-OH
Stéaroyl-Lys-Val-Dab-OH
Stéaroyl-Lys-Val-Dap-OH
Oléolyl-Lys-Val-Lys-OH
Palm-Lys-Val-Dab-OMe
Palm-Lys-Val-Dab-OOctyl
Palm-Lys-Val-Dab-OCétyl
Palm-Lys-Val-Dab-NH₂
Palm-Lys-Val-Dab-NHBu
Palm-Lys-Val-Dab-NHOctyl
Palm-Lys-Val-Dab-NHCétyl
Palm-Lys-Val-Dap-OMe
Palm-Lys-Val-Dap-OOctyl Palm-Lys-Val-Dap-OCétyl
Palm-Lys-Val-Dap-NH₂
Palm-Lys-Val-Dap-NHBu
Palm-Lys-Val-Dap-NHOctyl
Palm-Lys-Val-Dap-NHCétyl
C₁₄H₂₉-NH-CO-Lys-Val-Lys-OH
C₁₄H₂₉-NH-CO-Dab-Val-Dab-OH
C₁₄H₂₉-NH-CO-Lys-Ile-Dab-OH
C₁₄H₂₉-NH-CO-Lys-Val-Dap-OH
C₁₄H₂₉-NH-CO-Arg-Val-Arg-OH
C₁₄H₂₉-NH-CO-Dab-Val-Dap-OH
C₁₄H₂₉-NH-CO-Lys-Ile-Dap-OH
C₁₄H₂₉-NH-CO-Lys-Val-Dab-OH
C₁₄H₂₉-NH-CO-Dap-Val-Dap-OH
C₁₆H₃₃-NH-CO-Lys-Val-Lys-OH
C₁₈H₃₇-NH-CO-Lys-Val-Lys-OH
Boc-Lys-Val-Lys-OH
Ac-Lys-Val-Lys-OH
Z-Lys-Val-Lys-OH
Fmoc-Lys-Val-Lys-OH
C₈H₁₇-SO₂-Lys-Val-Lys-OH
C₁₆H₃₃-SO₂-Lys-Val-Lys-OH
H-Lys-Val-Lys-NH₂
H-Lys-Val-Lys-OOctyl
H-Lys-Val-Lys-OCétyl
H-Lys-Val-Lys-ORétinyl
H-Lys-Val-Lys-OTocophéryl
H-Lys-Val-Lys-OBn
H-Lys-Val-Lys-NH-Cétyl
H-Lys-Val-Lys-NH-Octyl
H-Lys-Val-Lys-NH-Bn
et leurs sels d'addition acides.

9. Méthode de fabrication des composés selon l'une des revendications 1-8, **caractérisée en ce qu'**on synthétise le composé de formule I dans sa totalité en appliquant des méthodes connues en chimie des peptides, qu'on scinde éventuellement- le(s) groupement(s) protecteur(s) restant et qu'on acyle ou sulfonyle éventuellement un groupement amino libre et/ou qu'on transforme le composé obtenu en sel d'addition acide et/ou un sel d'addition acide obtenu en la base conjuguée correspondante ou en un autre sel.

10. Utilisation des composés selon l'une des revendications 1-8 en tant que principe actif cosmétique, en particulier pour accroître la production de collagène et/ou d'élastine dans la peau humaine et/ou pour retarder ou traiter le vieillissement cutané.

11. Utilisation des composés selon l'une des revendications 1-8 pour la fabrication de préparations actives sur le plan cosmétique, en particulier en tant que produits de soins cutanés, notamment pour empêcher la formation et l'aggravation des rides et contre toutes les conséquences du vieillissement cutané naturel (endogène) ou accéléré (exogène).

12. Préparation cosmétique active comprenant au moins un composé selon l'une des revendications 1-8, de préférence en une quantité variant entre 0,5 ppm et 1000 ppm (p/p), de préférence entre 1 ppm et 100 ppm (p/p), calculée sur le poids du composé de la présente invention et du (ou des) véhiculeur(s).

13. Composition cosmétique active **caractérisée en ce qu'**elle contient :
a) au moins un dérivé tripeptidique de formule générale (I) selon l'une des revendications 1-8, et
b) une quantité sûre et efficace d'au moins un autre principe actif cutané sélectionné parmi les produits de desquamation, les agents anti-acnéiques, les composés de vitamine B3, les rétinoïdes, les di-, tri-, tétra- et pentapeptides ainsi que leurs dérivés, les hydroxy acides, les capteurs de radicaux, les agents anti-inflammatoires, les actifs bronzants, les agents cutanés blanchissants, les actifs anti-cellulitiques, les flavonoïdes, les actifs antimicrobiens, les agents cicatrisants, les actifs antimycosiques, les filtres et écrans solaires, le farnesol, le phytantriol, l'allantoïne, la glucosamine et leurs mélanges.

14. Préparation de soins pour la peau selon la revendication 13, comprenant un véhiculeur bien toléré par la peau.

15. Composition selon l'une des revendications 12-14, **caractérisée en ce qu'**elle se présente sous forme de solution, de dispersion, d'émulsion ou encapsulée dans des véhiculeurs, de préférence dans des macro-, micro- ou nanocapsules, dans des liposomes ou des chylomicrons, ou emprisonnée dans des macro-, micro- ou nanoparticules ou dans des micro-éponges ou absorbée sur des polymères organiques en poudre, du talc, de la bentonite ou d'autres véhiculeurs minéraux.

16. Composition selon l'une des revendications 12-14, **caractérisée en ce qu'**elle se présente sous forme d'émulsion, de lait, de lotion, d'onguent, de polymère gélifié, visqueux, tensio-actif et émulsifiant, de pommade, de shampooing, de savon, de gel, de poudre, de stick ou de crayon, de spray, d'huile corporelle, de masque facial ou de patch pour application transdermique.

17. Composition selon l'une des revendications 12-16 en tant que principe actif cosmétique, en particulier pour accroître la production de collagène et d'élastine dans la peau humaine, comme produit de soins cutanés, en particulier pour empêcher la formation et l'aggravation de rides et contre toutes les conséquences du vieillissement cutané naturel (endogène) ou accéléré (exogène).

18. Méthode cosmétique destinée à accroître la production de collagène et/ou d'élastine dans la peau humaine et/ou à retarder ou traiter le vieillissement cutané, **caractérisée en ce qu'**on applique sur la peau un composé selon l'une des revendications 1-8.
